# EUROPEAN PATENT SPECIFICATION

(11) **EP 2 773 743 B1**
(45) Date of publication and mention of the grant of the patent: **22.05.2019**
(21) Application number: 12845192.9
(22) Date of filing: 05.11.2012
(51) Int. Cl.: C12M 1/34

(54) **AFFINITY METHODS AND COMPOSITIONS EMPLOYING ELECTRONIC CONTROL OF PH**
AFFINITÄTSVERFAHREN UND -ZUSAMMENSETZUNGEN MIT ELEKTRONISCHER PH-STEUERUNG
PROCÉDÉS ET COMPOSITIONS BASÉS SUR L'AFFINITÉ FAISANT APPEL À LA RÉGULATION ÉLECTRONIQUE DU PH

(30) Priority: 04.11.2011 US 201161555630 P; 04.11.2011 US 201161555713 P
(43) Date of publication of application: 10.09.2014
(73) Proprietor: Bio-Rad Laboratories, Inc., Hercules, CA 94547 (US); Technion Research & Development Foundation Ltd., 32000 Haifa (IL)
(72) Inventor: PAULUS, Aran, Hercules, California 94547 (US); BOGOEV, Roumen, Hercules, California 94547 (US); ZAFIR-LAVIE, Inbal, Haifa 32000 (IL); DIGES, Camille, Hercules, California 94547 (US); BANDHAKAVI, Sricharan, Hercules, California 94547 (US); HAHN-WINDGASSEN, Annett, Hercules, California 94547 (US); POSCH, Anton, 85567 Grafting (DE); BROD, Elad, Haifa 32000 (IL); SIVAN, Uri, Haifa 32000 (IL)
(74) Representative: dompatent von Kreisler Selting Werner - Partnerschaft von Patent- und Rechtsanwälten mbB
(86) International application number: PCT/US2012/063502
(87) International publication number: WO 2013/067477

(56) References cited:
- WO-A1-01/36449
- US-A- 5 082 548
- US-A- 5 160 594
- US-A1- 2003 083 823
- US-A1- 2003 168 576
- US-A1- 2005 087 445
- US-A1- 2011 195 527
- O'NEILL ROGER A ET AL: "Isoelectric focusing technology quantifies protein signaling in 25 cells", PROCEEDINGS OF THE NATIONAL ACADEMY OF SCIENCES, NATIONAL ACADEMY OF SCIENCES, US, vol. 103, no. 44, 31 October 2006 (2006-10-31), pages 16153-16158, XP002517365, ISSN: 0027-8424, DOI: 10.1073/PNAS.0607973103
- KLYOHITO SHIMURA ET AL: "AFFINITY PROBE CAPILLARY ELECTROPHORESIS: ANALYSIS OF RECOMBINANT HUMAN GROWTH HORMONE WITH A FLUORESCENT LABELED ANTIBODY FRAGMENT", ANALYTICAL CHEMISTRY, AMERICAN CHEMICAL SOCIETY, vol. 66, no. 1, 1 January 1994 (1994-01-01), pages 9-15, XP000426220, ISSN: 0003-2700, DOI: 10.1021/AC00073A004
- KNITTLE JAMES E ET AL: "Laser-induced fluorescence detector for capillary-based isoelectric immunoblot assay", ANALYTICAL CHEMISTRY, AMERICAN CHEMICAL SOCIETY, vol. 79, no. 24, 15 December 2007 (2007-12-15), pages 9478-9483, XP002517551, ISSN: 0003-2700, DOI: 10.1021/AC071537Z [retrieved on 2007-11-17]
- A. J. HUGHES ET AL: "Microfluidic integration for automated targeted proteomic assays", PROCEEDINGS OF THE NATIONAL ACADEMY OF SCIENCES, vol. 109, no. 16, 17 April 2012 (2012-04-17), pages 5972-5977, XP055155248, ISSN: 0027-8424, DOI: 10.1073/pnas.1108617109

## Description

### BACKGROUND OF THE INVENTION

Detection of target molecules is useful in many industries. For example, detection and quantification of biological molecules is a basis for disease diagnostics. For instance, US 2011/195527; and the related scientific papers of O' Neill, R. A. et al., DNAS 103(44): 16153-158 (2006) and of Knittle, J. E. et al., Anal. Chem. 79 (24): 9478-83 (2007) disclose a method of target analyte detection employing a capillary covered with photoactivatable capture agents. Along the capillary a pH gradient is established and an electrical field is applied after sample loading to carry out IEF of the analyte. Next, the analyte is immobilized by photoactivation of the capture agent. This is followed by washing out the pH gradient by pulling out through a washing solution of a certain pH with the help of a vacuum source. For analyte detection, an affinity label is pulled through the capillary afterwards, equally by application of a vacuum. The pH gradient is established by two solutions having a different pH value at each end of the electric field with a gradient range of pH in between, ie a first ion injector. The vacuum pump in combination with the washing out solution is considered to be a second ion injector. Further Shimura, K. and Karger, B. L., Anal. Chem. 66(1): 9-15 (1994) disclose an analytical procedure in which samples are mixed with a purified labeled antibody target and the associated duplex is separated by capillary isoelectric focusing with detection by laser-induced Fluorescence.

### BRIEF SUMMARY OF THE INVENTION

Methods of detecting a target analyte are provided. the method comprising: providing into a chamber a sample comprising a mixture of molecules including one or more target analytes; the chamber comprising a first and a second electrode, wherein a first and second sub-areas of the compartment are between the electrodes; generating a pH gradient in the chamber with one or more proton and/or hydroxyl injector(s), applying a current across the chamber, thereby positioning analytes in the chamber at the first sub-area based on the isoelectric point (pI) of the analytes, wherein the target analyte is precipitated and/or adhered to the chamber once positioned at the first sub-area in the chamber corresponding to the target analyte pI; contacting the target analyte with a labeled affinity agent that specifically binds the target analyte;
applying a current between the first and second electrode thereby moving the affinity agent that bound to the target analyte, if any, to a second sub-area of the compartment, wherein solution in proximity to the second sub-area has a pH controlled by one or more proton/hydroxide injector(s); and
detecting the precipitated analyte.

The precipitated/adhered analyte may be captured in or adjacent to an opening (e.g., a slit) in the surface of the chamber. The detecting comprises contacting the precipitated analyte with an affinity agent that specifically binds the analyte. In some embodiments, the affinity agent is an antibody. In some embodiments, the binding of the affinity agent to the target analyte is detected by contacting the bound affinity agent with a secondary antibody and subsequently detecting the presence of the secondary antibody with a detectable label.

In some embodiments, the target analyte is a protein. In some embodiments, the target analyte is a post-translationally-modified protein.

| | | | | |
|---|---|---|---|---|
| **[0006]** | **[0007]** | **[0008]** | **[0009]** | **[0010]** |
| **[0011]** | **[0012]** | **[0013]** | **[0014]** | **[0015]** |
| **[0016]** | **[0017]** | **[0018]** | **[0019]** | **[0020]** |
| **[0021]** | **[0022]** | **[0023]** | **[0024]** | **[0025]** |
| **[0026]** | **[0027]** | **[0028]** | **[0029]** | **[0030]** |
| **[0031]** | **[0032]** | **[0033]** | **[0034]** | **[0035]** |
| **[0036]** | **[0037]** | | | |

In some embodiments, the affinity agent is an antibody.

The above described method provides for the detecting for presence, absence, or quantity of a target analyte in a sample, wherein the method comprises
providing a chamber comprising a first and a second electrode, wherein a first and second sub-area of the compartment are between the electrodes;
localizing components of a biological sample to the first sub-area of a compartment;
contacting the localized components with a labeled affinity agent that specifically binds the target analyte, if present, under conditions such that the affinity agent binds the target analyte, if present;
applying a current between the first and second electrode thereby moving the affinity agent that bound to the target analyte, if any, to a second sub-area of the compartment, wherein solution in proximity to the second sub-area has a pH controlled by one or more proton/hydroxide injector(s); and
detecting the presence or quantity of the affinity agent bound to the target analyte at the second sub-area of the compartment, thereby detecting the presence or quantity of a target analyte in a sample.

The affinity agent is an antibody.

The method may further comprise removing labeled affinity agent that is not bound to the target analyte prior to the applying of the current.

Labeled affinity agent not bound to the target analyte may not be removed prior to the applying of the current. The current moves the labeled affinity agent not bound to the target analyte to a third sub-area of the compartment, wherein solution in proximity to the third sub-area has a pH at approximately the pI of the labeled affinity agent not bound to the target analyte, wherein the pH of the solution in proximity to the third sub-area is controlled by one or more proton/hydroxide injector(s); and detecting the presence or quantity of the labeled affinity agent not bound to the target analyte. The method may comprise further determining the ratio of affinity agent bound to the target analyte at the second sub-area of the compartment and the quantity of the labeled affinity agent not bound to the target analyte at the third sub-area of the compartment.

The affinity agent may continue to bind the target analyte when moved to the second sub-area and wherein the pH of the solution in proximity to the second sub-area is approximately the pI of the affinity agent bound to the target analyte.

Between the contacting and the applying, the method may further comprise washing away affinity agent not bound to the target and then eluting the affinity agent bound to the target analyte such that the affinity agent no longer binds the target when moved to the second sub-area. The pH of the solution in proximity to the second sub-area may approximately the pI of the affinity agent not bound to the target analyte. The eluting may comprise changing the pH of solution in proximity to the first sub-area. The target analyte may be moved to a third sub-area of the compartment, wherein solution in proximity to the third sub-area has a pH at approximately the pI of the unbound target analyte, wherein the pH is controlled by one or more proton/hydroxide injector(s). The method may further comprise collecting the target analyte.

The localizing may comprise binding the components of the sample to the first sub-area of a compartment. The components may be bound directly to the first sub-area. Alternatively, the components may be bound indirectly to the first sub-area via one or more binding components. In particular, the components may be bound indirectly to the first sub-area via an antibody linked to the first sub-area. The components may be biotinylated and may be bound to the first sub-area by avidin or streptavidin linked to the first sub-area.

The chamber may be smaller at the second sub-area compared to the first sub-area.

The localizing may comprise applying a current, thereby moving charged components of the sample in proximity to the first sub-area.

The contacting may comprise contacting the localized components with a first and a second labeled affinity agent. In particular, the first and second affinity agents may have affinity for different target analytes and may have different pIs. The detecting may comprise detecting the presence or quantity of the first and second labeled affinity agent at different sub-areas distinguished by different pH of the solution in proximity to the different sub-areas, thereby localizing the first and second affinity agents by the affinity agents' different pIs. Furthermore, the first and second affinity agents may have affinity for different target analytes and have different detectable labels. The contacting may comprise greater than two labeled affinity agents with affinity towards different target analytes and have different detectable labels.

| | | | | | |
|---|---|---|---|---|---|
| **[0049]** | **[0050]** | **[0051]** | **[0052]** | **[0053]** | **[0054]** |
| **[0049]** | **[0056]** | **[0057]** | **[0058]** | **[0059]** | **[0060]** |
| **[0061]** | **[0062]** | **[0063]** | | | |

### BRIEF DESCRIPTION OF THE DRAWINGS

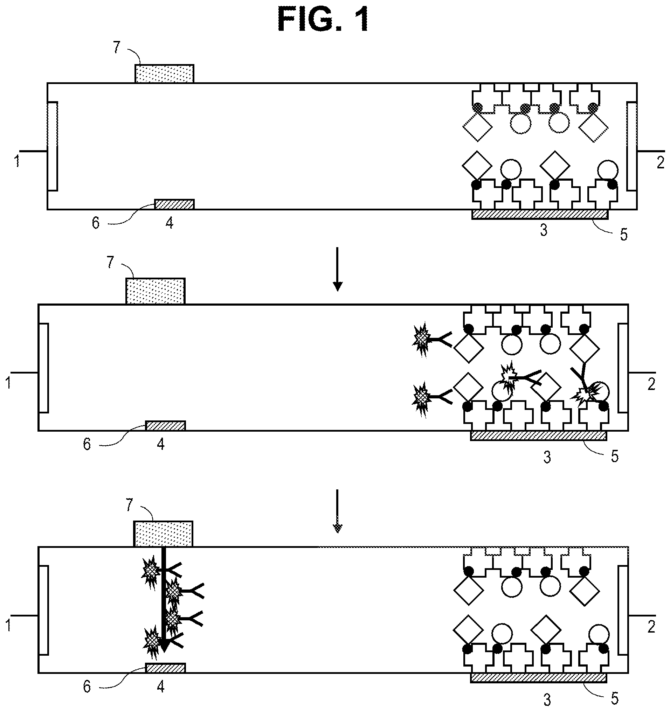

Figure 1 schematically illustrates an apparatus configuration and its use to detect a target molecule (represented by diamonds). At the top, the figure shows an apparatus in a first time point. The plus symbols represent streptavidin, and the circles and diamonds

In some embodiments, the target analyte is a post-translationally-modified protein.

Also provided is a method of capturing a target analyte from a mixture. In some embodiments, the method comprises, providing into a chamber a sample comprising a mixture of molecules including one or more target analyte, wherein one or more affinity specific for the target analyte is bound to a position on the chamber; generating a pH gradient in the chamber with one or more proton and/or hydroxide injector, thereby positioning analytes in the chamber based on the isoelectric point (pI) of the analytes, wherein the target analyte is positioned at its pI in proximity to the bound affinity agent(s), under conditions such that the target analyte is bound to the affinity agent(s).

In some embodiments, the method further comprises changing the pH gradient once the target analyte is in proximity to the bound affinity agent(s), thereby promoting conditions for binding.

In some embodiments, the method further comprises detecting the presence or absence of the target analyte.

In some embodiments, the method further comprises collecting the target analyte.

In some embodiments, the mixture comprises a sufficient amount of a non-ionic detergent or other agent (including but not limited to an organic solvent(s)) to promote solubility of the target analyte.

In some embodiments, the target analyte is a protein. In some embodiments, the affinity agent is an antibody.

Also provided is a device for capturing a target analyte from a mixture. In some embodiments, the device comprises a chamber for containing a solution having a plurality of molecular analytes along an axis, wherein the chamber comprises one or more opening (e.g., slit) in the surface of the chamber for collection of precipitated target analyte; an electrical source for applying an electric field along the axis in the chamber; a one or more ion sources for establishing a pH gradient along said axis in said chamber by injecting ion flows, capable of forming one or more pH steps in a pH gradient; a controller which operates said one or more ion sources to adjust the pH gradient so as to induce migration of the molecular analytes separately along the axis.

Also provided is a device for capturing a target analyte from a mixture. In some embodiments, the device comprises a chamber for containing a solution having a plurality of molecular analytes along an axis, wherein one or more affinity agents are bound to the interior surface of the chamber at a position on the chamber; an electrical source for applying an electric field along the axis in the chamber; a one or more ion sources for establishing a pH gradient along said axis in said chamber by injecting ion flows, capable of forming one or more pH steps in a pH gradient; a controller which operates said one or more ion sources to adjust the pH gradient so as to induce migration of the molecular analytes separately along the axis.

In some embodiments, the affinity agent is an antibody.

Methods of detecting for presence, absence, or quantity of a target analyte in a sample are also provided. In some embodiments, the method comprises
providing a chamber comprising a first and a second electrode, wherein a first and second sub-area of the compartment are between the electrodes;
localizing components of a biological sample to the first sub-area of a compartment;
contacting the localized components with a labeled affinity agent that specifically binds the target analyte, if present, under conditions such that the affinity agent binds the target analyte, if present;
applying a current between the first and second electrode thereby moving the affinity agent that bound to the target analyte, if any, to a second sub-area of the compartment, wherein solution in proximity to the second sub-area has a pH controlled by one or more proton/hydroxide injector(s); and
detecting the presence or quantity of the affinity agent bound to the target analyte at the second sub-area of the compartment, thereby detecting the presence or quantity of a target analyte in a sample.

In some embodiments, the affinity agent is an antibody.

In some embodiments, the method further comprises removing labeled affinity agent that is not bound to the target analyte prior to the applying of the current.

In some embodiments, labeled affinity agent not bound to the target analyte is not removed prior to the applying of the current. In some embodiments, the current moves the labeled affinity agent not bound to the target analyte to a third sub-area of the compartment, wherein solution in proximity to the third sub-area has a pH at approximately the pI of the labeled affinity agent not bound to the target analyte, wherein the pH of the solution in proximity to the third sub-area is controlled by one or more proton/hydroxide injector(s); and detecting the presence or quantity of the labeled affinity agent not bound to the target analyte. In some embodiments, the method comprises further determining the ratio of affinity agent bound to the target analyte at the second sub-area of the compartment and the quantity of the labeled affinity agent not bound to the target analyte at the third sub-area of the compartment.

In some embodiments, the affinity agent continues to bind the target analyte when moved to the second sub-area and wherein the pH of the solution in proximity to the second sub-area is approximately the pI of the affinity agent bound to the target analyte.

In some embodiments, between the contacting and the applying, further comprising washing away affinity agent not bound to the target and then eluting the affinity agent bound to the target analyte such that the affinity agent no longer binds the target when moved to the second sub-area. In some embodiments, the pH of the solution in proximity to the second sub-area is approximately the pI of the affinity agent not bound to the target analyte. In some embodiments, the eluting comprises changing the pH of solution in proximity to the first sub-area. In some embodiments, the target analyte is moved to a third sub-area of the compartment, wherein solution in proximity to the third sub-area has a pH at approximately the pI of the unbound target analyte, wherein the pH is controlled by one or more proton/hydroxide injector(s). In some embodiments, the method further comprise collecting the target analyte.

In some embodiments, the localizing comprises binding the components of the sample to the first sub-area of a compartment. In some embodiments, the components are bound directly to the first sub-area. In some embodiments, the components are bound indirectly to the first sub-area via one or more binding components. In some embodiments, the components are bound indirectly to the first sub-area via an antibody linked to the first sub-area. In some embodiments, the components are biotinylated and are bound to the first sub-area by avidin or streptavidin linked to the first sub-area.

In some embodiments, the chamber is smaller at the second sub-area compared to the first sub-area.

In some embodiments, the localizing comprises applying a current, thereby moving charged components of the sample in proximity to the first sub-area.

In some embodiments, the contacting comprises contacting the localized components with a first and a second labeled affinity agent. In some embodiments, the first and second affinity agents have affinity for different target analytes and have different pIs. In some embodiments, the detecting comprises detecting the presence or quantity of the first and second labeled affinity agent at different sub-areas distinguished by different pH of the solution in proximity to the different sub-areas, thereby localizing the first and second affinity agents by the affinity agents' different pIs. In some embodiments, the first and second affinity agents have affinity for different target analytes and have different detectable labels. In some embodiments, the contacting comprises greater than two labeled affinity agents with affinity towards different target analytes and have different detectable labels.

In some embodiments, the method comprises:
providing a chamber comprising a first and a second electrode on two different sides of the chamber, wherein a first and second sub-area of the compartment are between the electrodes;
localizing components of a sample to the first sub-area of a compartment;
contacting the localized components with an affinity agent under conditions such that the affinity agent specifically binds the target analyte, if present, wherein the affinity agent is linked to an enzyme;
removing affinity agent that is not bound to the target analyte prior to the applying;
contacting a substrate to the enzyme, thereby generating a detectable processed substrate;
applying a current between the first and second electrode thereby moving the detectable processed substrate, if any, to the second sub-area of the compartment, wherein solution in proximity to the second sub-area has a pH at approximately the pI of the processed substrate, wherein the pH is controlled by one or more proton/hydroxide injector(s); and
detecting the presence or quantity of the detectable processed substrate at the second sub-area of the compartment, thereby detecting the presence or quantity of a target analyte in a sample.

In some embodiments, the localizing comprises binding the components directly to the first sub-area.

In some embodiments, the localizing comprises indirectly binding the components to the first sub-area via one or more binding components. In some embodiments, the localizing comprises indirectly binding the components to the first sub-area via an antibody linked to the first sub-area. In some embodiments, the components are biotinylated and the localizing comprises binding the biotinylated components to the first sub-area by avidin or streptavidin linked to the first sub-area.

In some embodiments, the chamber is smaller at the second sub-area compared to the first sub-area.

In some embodiments, the affinity agent is an antibody.

In some embodiments, a primary antibody binds the component and the enzyme is directly linked to the primary antibody.

In some embodiments, a primary antibody binds the component, a second antibody binds the primary antibody and the enzyme is linked to the secondary antibody.

In some embodiments, the enzyme is selected from the group consisting of horseradish peroxidase (HRP), alkaline phosphatase, and luciferase.

In some embodiments, the method comprises:
providing a chamber comprising a first and a second electrode on two different sides of the chamber, wherein:
   a first and second sub-area of the compartment are between the electrodes;
   an affinity agent specific for the target molecule is linked to the first sub-area, and;
   the affinity agent is bound to a labeled competitor molecule, wherein the competitor competes with the target molecule for binding to the affinity agent;
contacting the affinity agent to a biological sample that may contain the target molecule under conditions such that the target molecule, if present in the biological sample, displaces the labeled target molecule or portion thereof from the affinity agent;
applying a current between the first and second electrode thereby moving the displaced labeled target molecule or portion thereof, if any, to a second sub-area of the compartment, wherein solution in proximity to the second sub-area has a pH at approximately the pI of the displaced labeled target molecule or portion thereof, wherein the pH is controlled by one or more proton/hydroxide injector(s); and
detecting the presence or quantity of the displaced labeled target molecule or portion thereof at the second sub-area of the compartment, wherein the presence or quantity of the displaced molecule or portion thereof corresponds to the presence or quantity of the target analyte in the biological sample, thereby detecting the presence or quantity of a target analyte in a sample.

In some embodiments, the labeled competitor molecule is a labeled target molecule, or a labeled portion thereof.

In some embodiments, the affinity agent is an antibody.

In some embodiments, the chamber is smaller at the second sub-area compared to the first sub-area.

Also provided is an apparatus. In some embodiments, the apparatus comprises: a chamber comprising a first and a second electrode, wherein a first and second sub-area of the compartment are between the electrodes, wherein affinity agents (e.g., antibodies, avidin, streptavidin, etc.) are linked to the first sub-area; and the second sub-area comprises a fluorescence detector and one or more proton/hydroxide injector(s).

In some embodiments, the chamber further comprises a third sub-area between the electrodes and the third sub-area comprises one or more further proton/hydroxide injector(s). In some embodiments, the third sub-area further comprises a further fluoresce detector. In some embodiments, the affinity agents are antibodies.

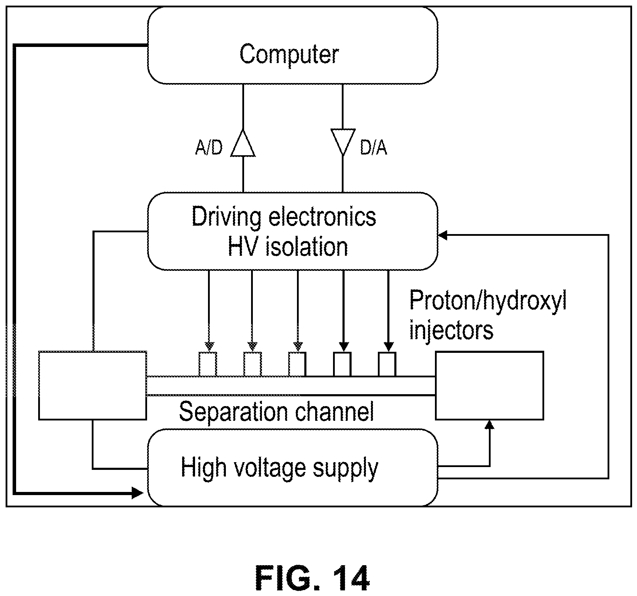

Also provided is a system comprising an apparatus as described above or elsewhere herein (e.g., a chamber comprising a first and second electrode, one or more proton injector and/or hydroxide injector at a first sub-area of the chamber, optionally one or more affinity agents linked to a second sub-area of the chamber, optionally one or more outlet in the chamber, e.g., for sample addition or collection) and a power source (see, e.g., Figure 14) for controlling the current or voltage between the first and second electrodes and/or the proton or hydroxide injectors. In some embodiments, the system further comprises a pump (e.g., for pumping fluid from or to the chamber and/or for pumping fluid through one or more injector). In some embodiments, the system further comprises a heating or cooling unit, e.g., for maintaining temperature of the fluid in the chamber and/or in one or more injectors. In some embodiments, the system comprises a stir bar or other mixing apparatus for mixing fluid in the chamber. In some embodiments, the system, comprises a pH meter and/or ionic strength meter.

### BRIEF DESCRIPTION OF THE DRAWINGS

Figure 1 schematically illustrates an apparatus configuration and its use to detect a target molecule (represented by diamonds). At the top, the figure shows an apparatus in a first time point. The plus symbols represent streptavidin, and the circles and diamonds represent components of a sample, with the diamonds being the target molecule. The smaller filled circles represent biotin moieties on the sample components. The middle section of the figure illustrates the apparatus at a second time point with addition of a labeled affinity agent specific for the target molecule. The bottom section of the figure illustrates a third time point following a wash and subsequent elution of the detectably-labeled affinity agents and movement of the detectably-labeled affinity agents to a second sub-area (4) of the apparatus. Movement of the detectably-labeled affinity agent can be achieved, for example, by setting the pH in the first sub-area (3) to a pH different than the pI of the detectably-labeled affinity agent such that the detectably-labeled affinity agent has a charge. The pH of the solution in proximity to the first sub-area (3) can be controlled by an ion injector (5). The electrodes (1, 2) can then generate a field in which the charged detectably-labeled affinity agent moves towards the second sub-area (4). Item 6 represents an ion injector that can control the pH in proximity to the second sub-area (4). The ion injector (6) can generate a pH at or close to the pI of the detectably-labeled, such that when the detectably-labeled affinity agent is in proximity to the ion injector (6) the detectably-labeled affinity agent is no longer charged and therefore no longer moves in the electrical field. Once located in the second sub-area (4), the detectably-labeled affinity agents can be detected or quantified by a detector (7).

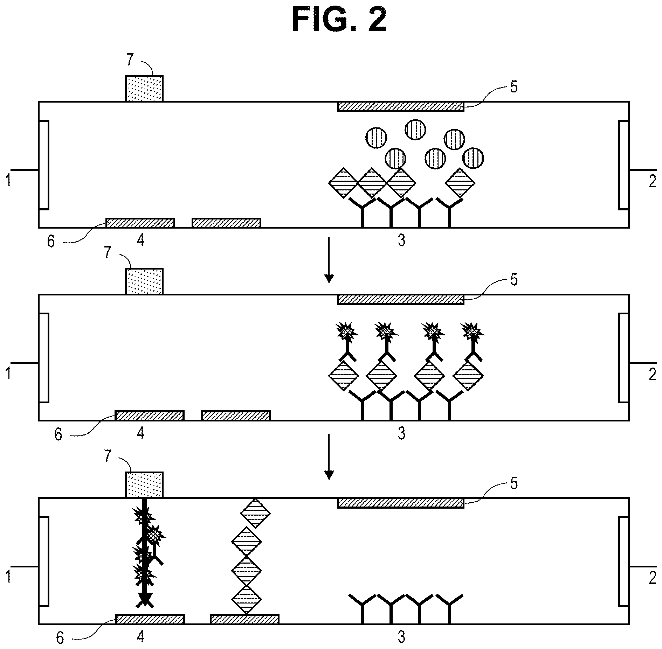

Figure 2 schematically illustrates an apparatus configuration and its use to detect a target molecule (represented by diamonds). At the top, the figure shows the sample is initially provided near a first sub-area (3) of the apparatus, where affinity agents are linked to the apparatus. The affinity agents specifically bind to the target molecules (diamonds) in the sample while not binding other components (represented by circles) of the sample. The middle section of the figure shows the addition of detectably-labeled affinity agents specific for the target molecule, acting in a sandwich-like format. The bottom section of the figure shows the apparatus following a wash and subsequent elution of the detectably-labeled affinity agents and movement of the detectably-labeled affinity agents to a second sub-area (4) of the apparatus. Movement of the detectably-labeled affinity agent can be achieved, for example, by setting the pH in the first sub-area (3) to a pH different than the pI of the detectably-labeled affinity agent such that the detectably-labeled affinity agent has a charge. The pH of the solution in proximity to the first sub-area (3) can be controlled by an ion injector (5). The electrodes (1, 2) can then generate a field in which the charged detectably-labeled affinity agent moves towards the second sub-area (4). Item 6 represents an ion injector that can control the pH in proximity to the second sub-area (4). The ion injector (6) can generate a pH at or close to the pI of the detectably-labeled affinity agent, such that when the detectably-labeled affinity agent is in proximity to the ion injector (6) the detectably-labeled affinity agent is no longer substantially charged and therefore no longer moves in the electrical field. Once located in the second sub-area (4), the detectably-labeled affinity agents can be detected or quantified by a detector (7).

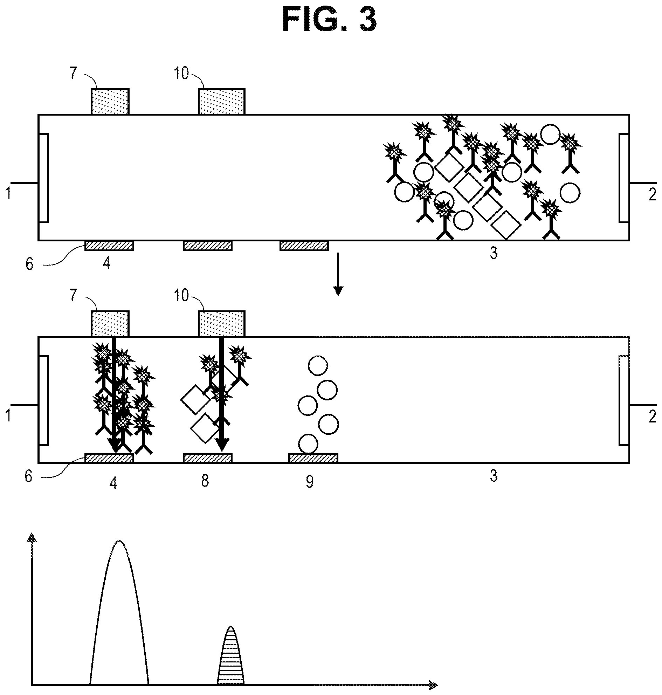

Figure 3 schematically illustrates an apparatus configuration and its use to detect a target molecule (represented by diamonds). At the top, the figure shows the sample (diamonds and unwanted sample components (circles)) is initially provided near a first sub-area (3) of the apparatus. Detectably-labeled affinity agents are also provided in contact with the sample, either having been mixed with the sample beforehand or added before or after the sample is added. The conditions allow for binding of the affinity agents to target molecules, if present in the sample. The middle section of the figure shows that the detectably-labeled affinity agents that did not bind a target molecule are moved to a second sub-area (4) of the apparatus different from a third sub-area (8) where detectably-labeled affinity agent bound to target molecules are located. Optionally, other components of the sample are localized to a fourth sub-area (9). Movement of these molecules (detectably-labeled affinity agent bound to target molecules, detectably-labeled affinity agent not bound to target molecules, optionally other sample components) is achieved as discussed above, i.e., using the electrodes to move charged molecules to a sub-area where the pH is close to or at the molecules' pI. Different detectors (7, 10) can be used to quantify the separate amounts of detectably-labeled affinity agent bound to target molecules and detectably-labeled affinity agent not bound to target molecules, wherein the ratio of these amounts can be used to determine the amount of target molecules in the sample. The bottom part of figure illustrates a prophetic quantitative analysis of amounts of detectably-labeled affinity agent bound to target molecules and detectably-labeled affinity agent not bound to target molecules.

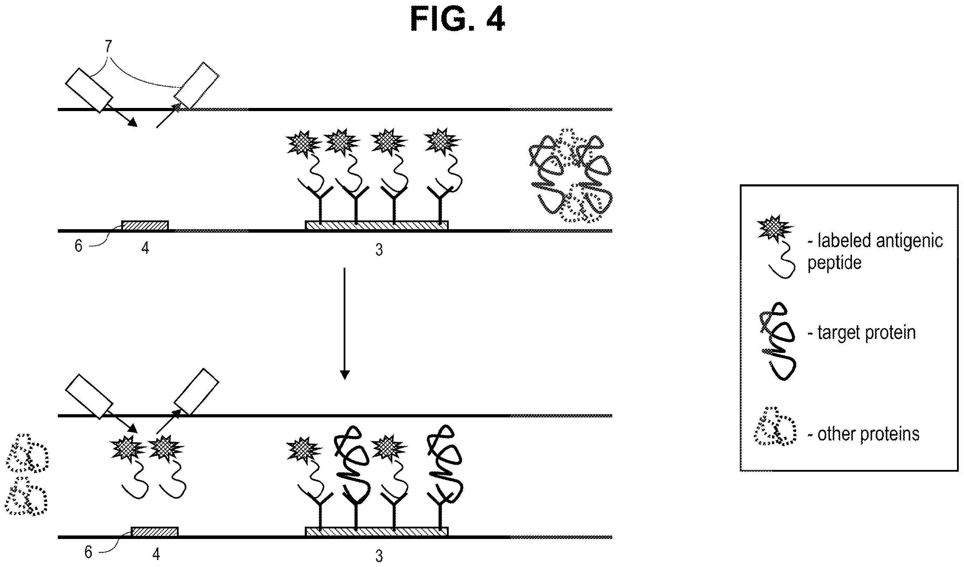

Figure 4 schematically illustrates an apparatus configuration and its use to detect a target molecule. At the top, the figure shows an initial time point at which a chamber in the apparatus comprises affinity agents for target molecules linked to a first chamber sub-area (3). Bound to these linked affinity agents are detectably-labeled molecules that compete for binding to the affinity agents with the target molecules. As shown in the right portion of the chamber, a sample is provided that contains a target molecule in addition to other molecules. Upon contact of the sample with the linked affinity agents, target molecules in the sample will compete and displace some amount of the detectably-labeled molecules on the affinity agents. The displaced detectably-labeled molecules can then be moved to a second sub-area (4) of the chamber (for example, using electrodes to generate an electrical field that moves charged detectably-labeled molecules, as described above and elsewhere herein) where one or more detector (7) is used to detect displaced detectably-labeled molecules. *See,* bottom part of figure. While not shown in the figure, proton or hydroxide injectors can be placed, for example, in proximity to the first (3) or second (4) sub-areas to set the pH such that, for instance, molecules to be moved with electrical fields are charged, or not, as appropriate to achieve their movement.

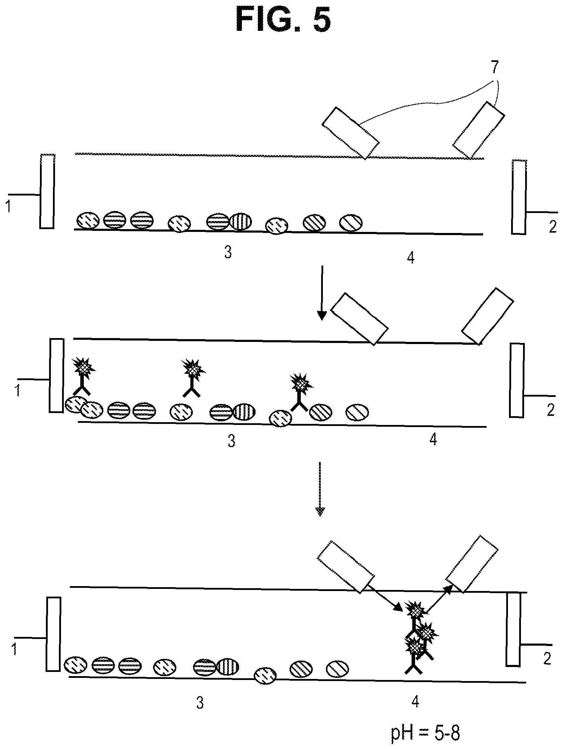

Figure 5 schematically illustrates an apparatus configuration and its use to detect multiple different target molecules. The top portion of the figure shows a complex sample composed of multiple different components (represented by ovals). The sample components are linked to a first sub-area (3) of the chamber of the apparatus. The middle portion of the figure illustrates the apparatus following addition of detectably-labeled affinity agents specific for two or more different target molecules. In the illustration, three different affinity agents are included, each binding a different target molecule. For example, the three targets could be three different types of proteins. The bottom portion of the figure illustrates a time point after non-binding affinity agents have been washed away and bound affinity agents are subsequently eluted. Optionally, elution can be achieved by changing the pH of the solution in the chamber, e.g., with proton or hydroxide injectors (not shown). The electrodes (1,2) on sides of the chamber can generate an electrical field, thereby moving charged molecules, including the eluted detectably-labeled affinity agents, to a second sub-area (4) of the chamber. The moved detectably-labeled affinity agents can be localized at the second sub-area by generating a pH at or close to the pI of the detectably-labeled affinity agents in the solution in proximity to the second sub-area (4), e.g., with one or more proton or hydroxide injector (not shown). For example, the pH in proximity to the second sub-area can be between 5-8. Once localized, the different detectably-labeled affinity agents can be individually detected, for example if the affinity agents for different targets comprise different detectable labels that can be distinguished (e.g., in the case of fluorescent labels, that emit signal at different wavelengths or that are excited at different wavelengths). The signal of the labels can be detected with a detector (7).

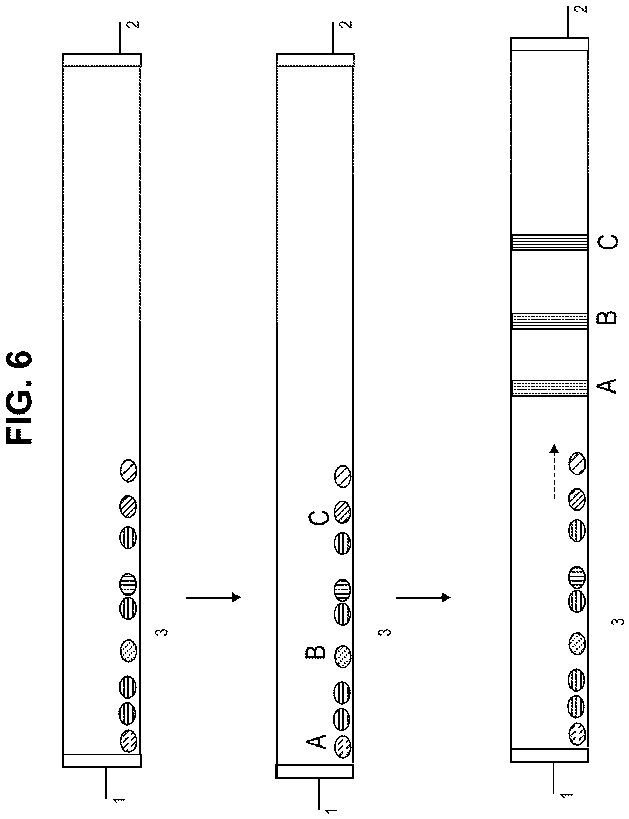

Figure 6 schematically illustrates an apparatus configuration and its use to detect multiple different target molecules. The top portion of the figure shows a complex sample composed of multiple different components (represented by ovals). The sample components are linked to a first sub-area (3) of the chamber of the apparatus. The middle portion of the figure illustrates the apparatus following addition of detectably-labeled affinity agents (A, B, C) specific for two or more different target molecules. In the illustration, three different affinity agents (A, B, C) are included, each binding a different target molecule. For example, the three targets could be three different types of proteins. The bottom portion of the figure illustrates a time point after non-binding affinity agents have been washed away and bound affinity agents are subsequently eluted. Optionally, elution can be achieved by changing the pH of the solution in the chamber, e.g., with proton or hydroxide injectors (not shown). The electrodes (1,2) on sides of the chamber can generate an electrical field, thereby moving charged molecules, including the eluted detectably-labeled affinity agents, to different sub-areas (A, B, C) of the chamber, each sub-area representing localization of a different affinity agent. This can be achieved, for example, where different detectably-labeled affinity agents have a different pI. Thus, by using a different proton or hydroxide injector(s) at different sub-areas to generate a localized pH at or close to the pI of different affinity agents, detection of signal at a particular sub-area should be generated only, or substantially only, from one particular affinity agent type. In this configuration, different detectable labels can be, but do not have to be, used by different affinity agents. The moved detectably-labeled affinity agents can be localized at the sub-areas by generating a pH at or close to the pI of the detectably-labeled affinity agents in the solution in proximity to the second sub-area (4), e.g., with one or more proton or hydroxide injector (not shown).

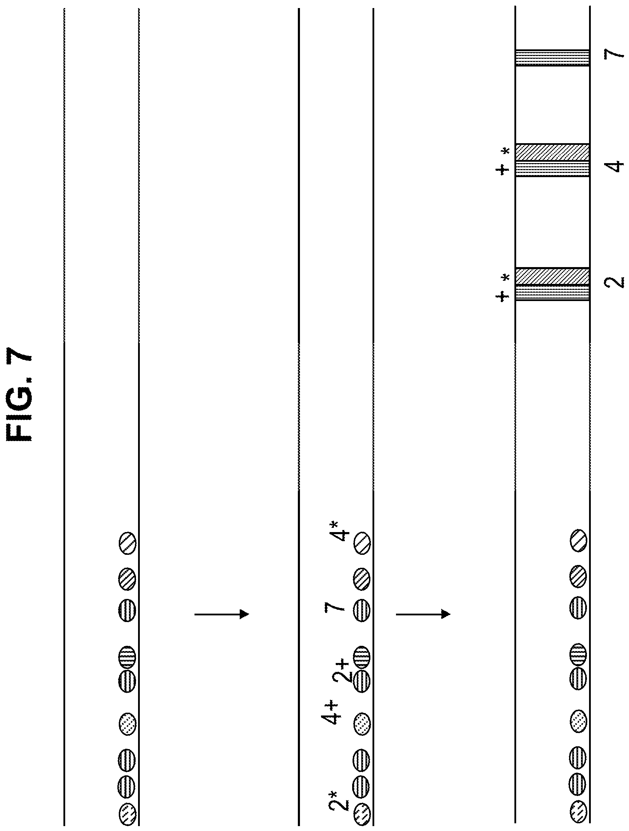

Figure 7 schematically illustrates an apparatus configuration and its use to detect multiple different target molecules. Figure 7 illustrates a configuration combining aspects shown in Figures 5 and 6. Thus, affinity agents are used, some of which can be distinguishable by pI with others distinguishable by the signal of the label. In this figure, affinity agents are represented by numbers (2*, 2+, 4*, 4+, 7) with asterisks (*) and plus signs (+) representing different affinity agents that have the same pI but different detectable labels. The numbers are intended to indicate exemplary pIs, i.e., affinity agents 2* and 2+ both have a pI around 2, affinity agents 4* and 4+ have a pI around 4, and affinity agent 7 has a pI around 7. Once the affinity agents are eluted from their respective targets (bottom part of figure), the affinity agents are moved by an electrical field to separate detection areas having different localized pHs (∼2, ∼4, and ∼7). In situations where more than one affinity agent has the same approximate pI (e.g., 2* and 2+), they are distinguished by their signals. Thus for example, to distinguish 2* and 2+, the two affinity agents must have distinguishable signals. However, there is no need for 2* and 7 to have different signals, because those affinity agents are localized to different detectors.

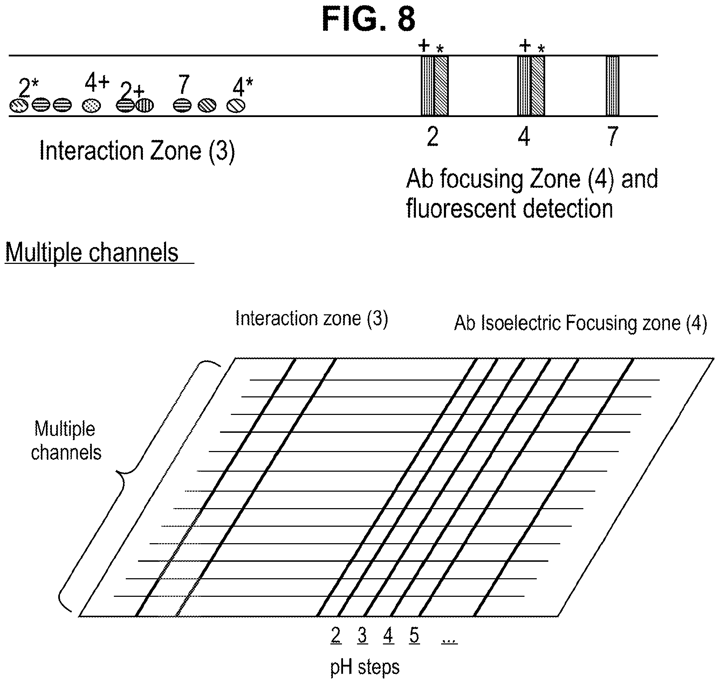

Figure 8 schematically illustrates an apparatus configuration and its use to detect multiple different target molecules similar to Figure 7. The bottom portion of the figure illustrates how that top portion can be configured in replicate channels, thereby allowing for multiple samples to be analyzed in parallel for multiple targets.

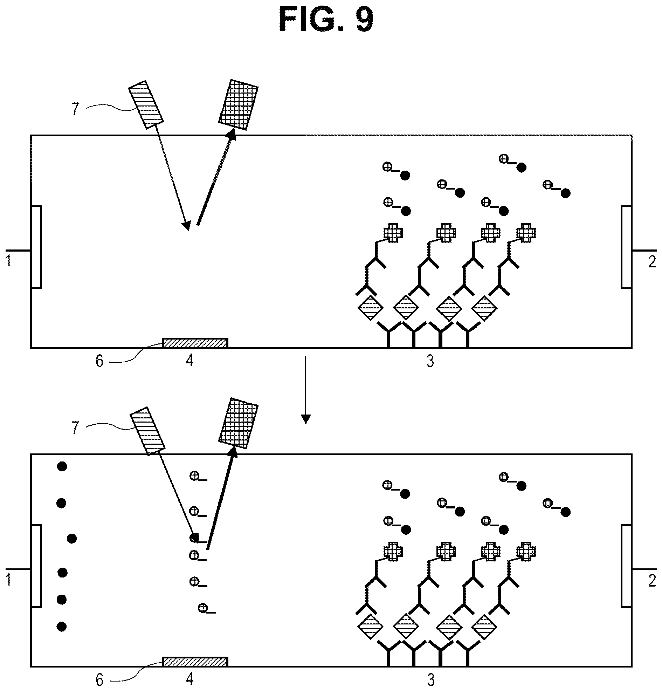

Figure 9 schematically illustrates an apparatus configuration and its use to detect target molecules from a sample. In the aspect illustrated in Figure 9, an affinity agent labeled with an enzyme that renders a substrate detectable is used. The apparatus comprises a chamber having an affinity agent specific for the target molecule linked to a first sub-area (3) of the chamber. The affinity agent acts to capture a target molecule (diamonds) from a sample. The capture target molecule can then be detected by addition of an affinity agent linked to an enzyme, or as depicted in Figure 9, a primary affinity agent specific for the captured target can be bound the target molecule and then a secondary affinity agent linked to an enzyme (plus sign) can be used to bind to the primary affinity agent. The substrate of the enzyme is shown as two circles linked by a line. The processed substrate (after acted upon by the enzyme) is shown as a dark circle and a light circle with a line, the latter representing the detectable processed substrate. At the bottom, the figure shows a later time point at which the processed substrate has been moved in an electrical field created by the electrodes (1, 2) in proximity to a second sub-area (4). The pH of the solution in proximity of the second sub-area (4) is set to a pH at or close to the pI of the detectable processed substrate by an proton or hydroxide injector (6). The detectable processed substrate can then be detected by a detector (7). This aspect can be performed in multiplex if desired by using affinity agents with different target specificity linked to different enzymes such that different detectable processed substrates can be distinguished (e.g., by wavelength, pI, or other criteria).

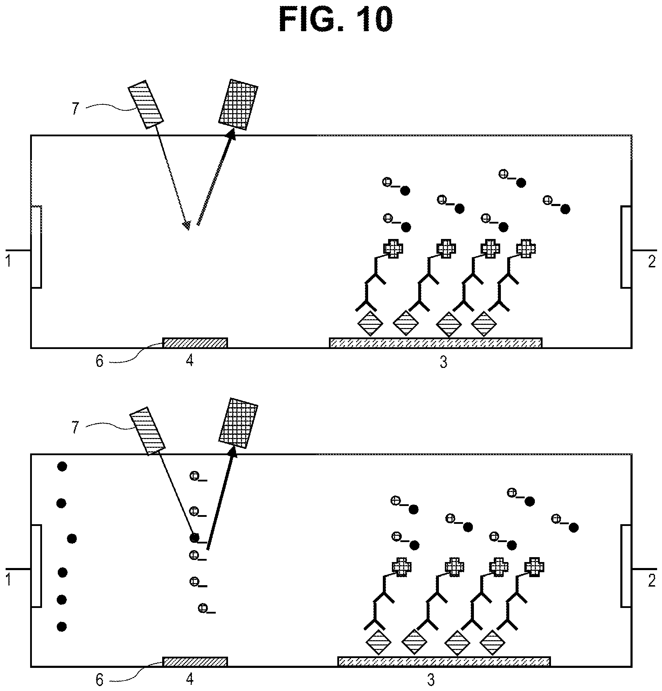

Figure 10 schematically illustrates an apparatus configuration and its use to detect target molecules from a sample. Figure 10 is similar to Figure 9, but instead of using a capture affinity agent, the target molecule(s) (and optionally other sample components) are linked directly or indirectly to the first sub-area of the chamber. The remaining aspects of the method are similar to those described for Figure 9. This aspect can be performed in multiplex if desired by using affinity agents with different target specificity linked to different enzymes such that different detectable processed substrates can be distinguished (e.g., by wavelength, pI, or other criteria).

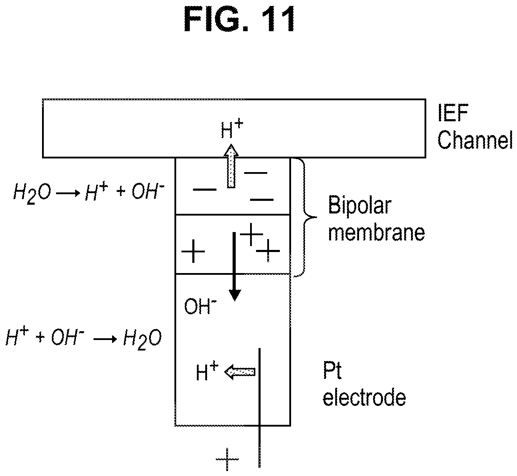

Figure 11 illustrates a proton injector comprising a small compartment adjacent to the chamber of the apparatus described herein, with an electrode (e.g., a Pt electrode) in contact with the solution in the compartment, and a bipolar membrane separating the compartment from the chamber. This figure is not intended to show the entire apparatus but merely shows how a proton injector can be attached to a chamber.

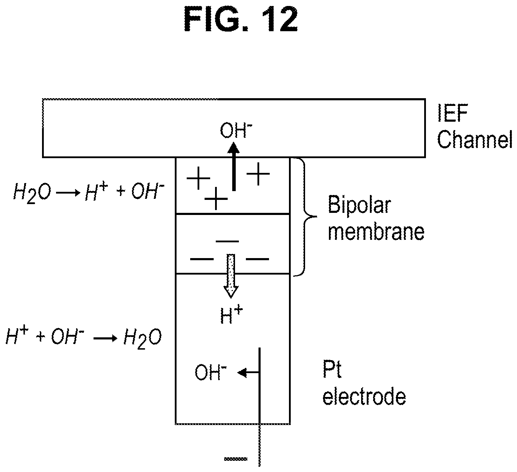

Figure 12 illustrates a hydroxide injector comprising a small compartment adjacent to the chamber of the apparatus described herein, with an electrode (e.g., a Pt electrode) in contact with the solution in the compartment, and a bipolar membrane separating the compartment from the chamber. This figure is not intended to show the entire apparatus but merely shows how a hydroxide injector can be attached to a chamber.

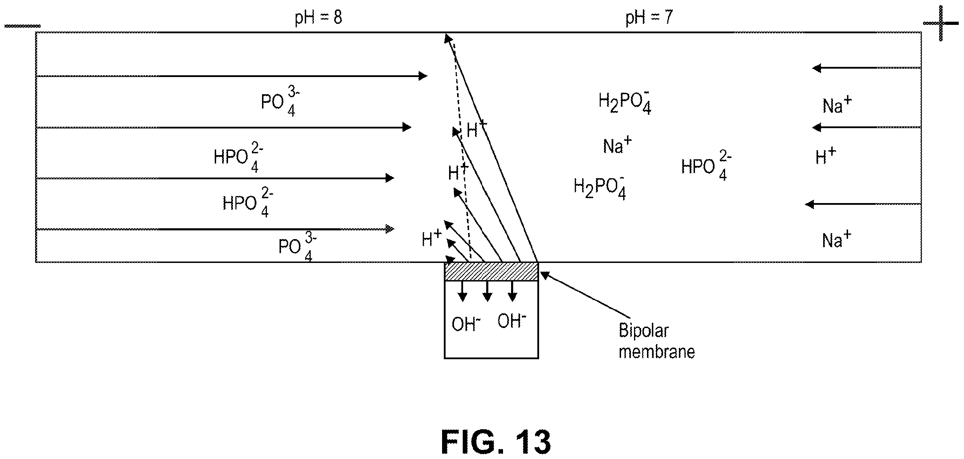

Figure 13 illustrates possible electrolytes and their interaction with a proton/hydroxide injector.
Figure 14 illustrates an embodiment for a system controlling a proton/hydroxide injector device.

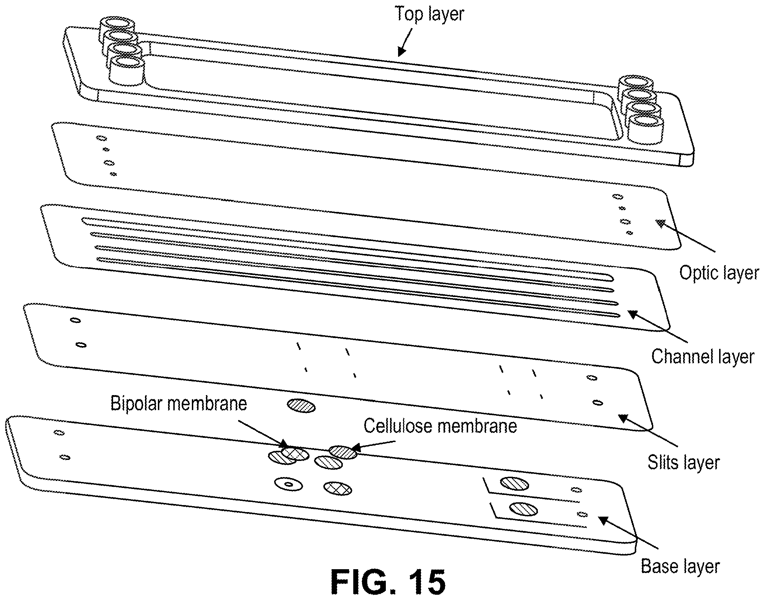

Figure 15 illustrates an embodiment of an integrated disposable channel for use in a proton/hydroxide injector device. Openings (e.g., slits) for fluid contact to proton/hydroxide injector compartments can be arranged as desired. For example, in some embodiments, slits in the chamber are 1-1000 microns, and in some embodiments, about 100 micron. The number and size of slits can be designed to generate step-wise pH gradients as desired. Cellulose, or other hydrophilic, membranes, for example, as shown in Figure 15 are optional, and function to cover unused slits and/or can optionally cover bipolar membranes to the extent sample components have affinity to the bipolar membrane. In some embodiments, instead of hydrophilic membranes, hydrophilic coatings may be used to cover the bipolar membranes and prevent binding of the sample components to it. Further openings (e.g., slit) can be used to extract and inject samples to the channel.

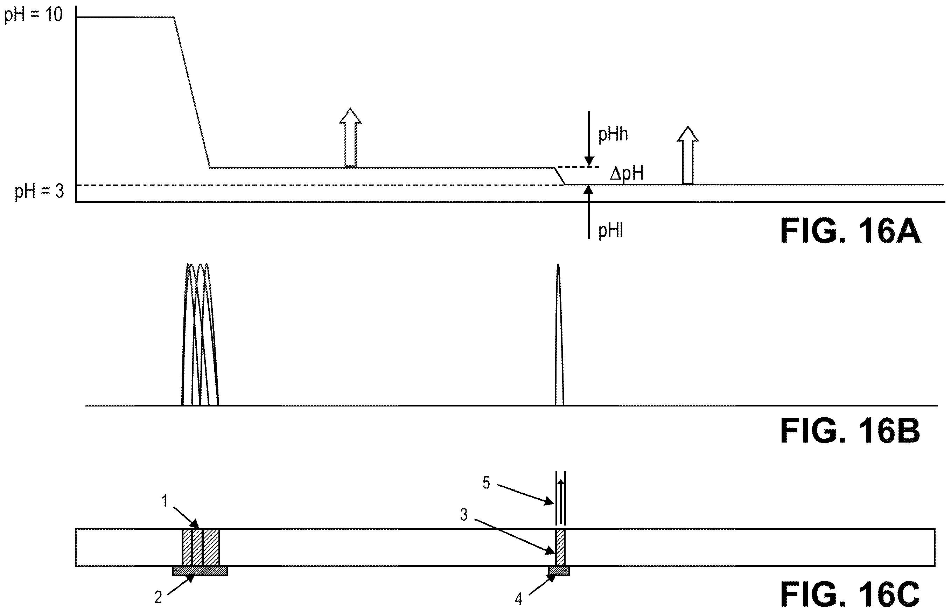

Figures 16A-C illustrate generation of a pH step gradient and isolation of target molecules with the gradient. In the figure, a bipolar membrane (2) generates a large pH differential, thereby focusing unwanted components (1) of the sample away from the target analyte. A second bipolar membrane (4) generates a small pH differential centered at the pI for the target analyte (3). The target analyte (3) can optionally be collected in a channel (5) in the chamber.

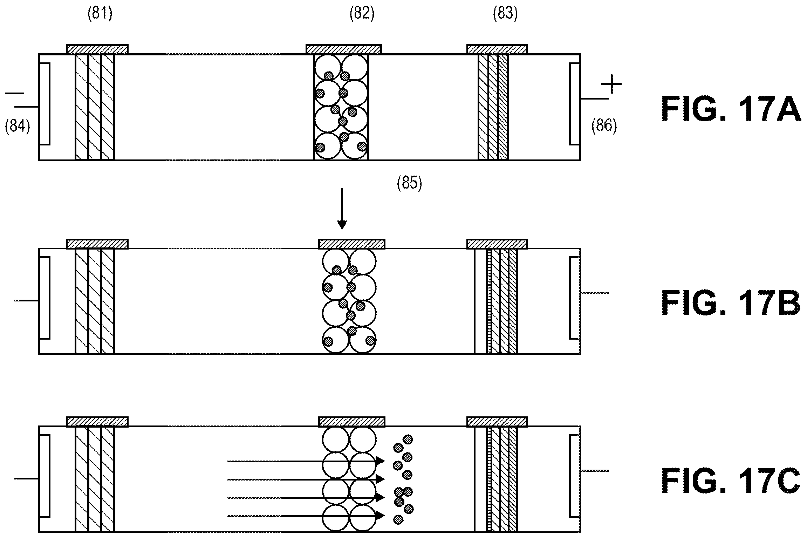

Figure 17A-C illustrates an embodiment in which a chamber comprises a solid support linked to affinity ligands (affinity agents). In Figure 17A, a target analyte (shown as dots) is positioned in the chamber in proximity to the solid support using pI focusing as described herein, thereby binding the target analyte to the affinity ligand. Figure 17B illustrates a subsequent stage in which the pH gradient is removed. Figure 17C illustrates an embodiment in which the target analyte is subsequently eluted from the affinity agent, e.g., for collection as a purified product. Elution can occur in any way desired. In some embodiments, the solution is changed to elute the analyte. In some embodiments, the proton/hydroxide ion injector is used to change the pH in proximity to the affinity ligands to a pH resulting in elution.

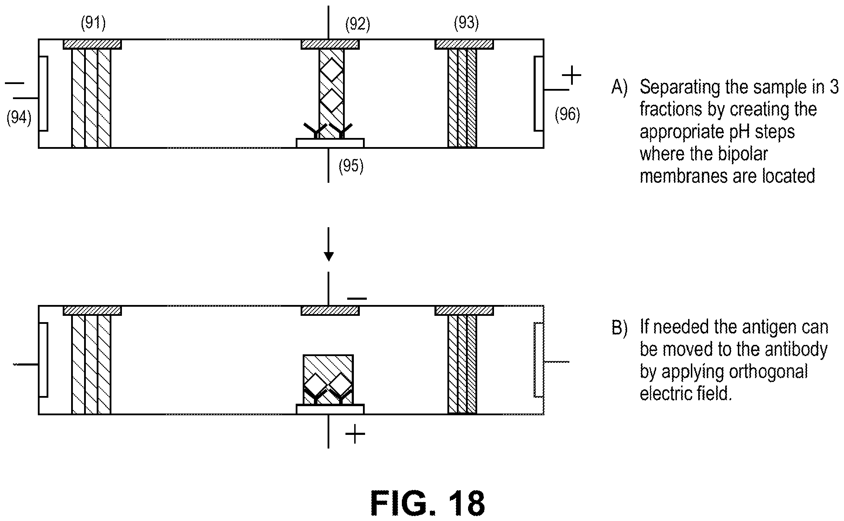

Figure 18 illustrates an embodiment in which a target analyte is focused to a specific area of the chamber based on pI, thereby locating the target analyte in proximity with antibodies (specific for the target analyte) adhered in the chamber. In some of these embodiments, the target analyte is not precipitated at its pI. In other embodiments, the target analyte is precipitated.

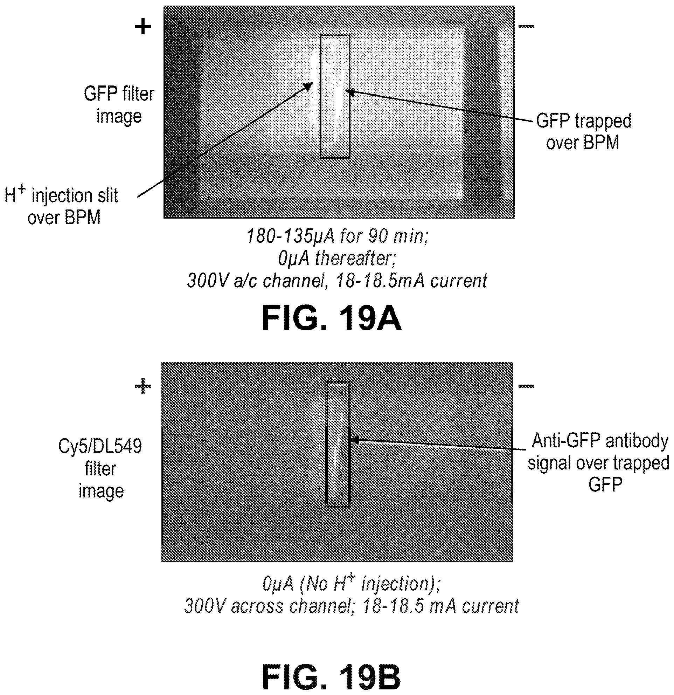

Figure 19A represents green fluorescent protein (GFP) signal following isoelectric focusing and adherence to chamber via prolonged H⁺ injection in buffer in a chamber. Figure 19B illustrates CY5 signal following precipitation/adherence of the GFP and electrophoretic introduction of a CY5-labeled anti-GFP antibody.

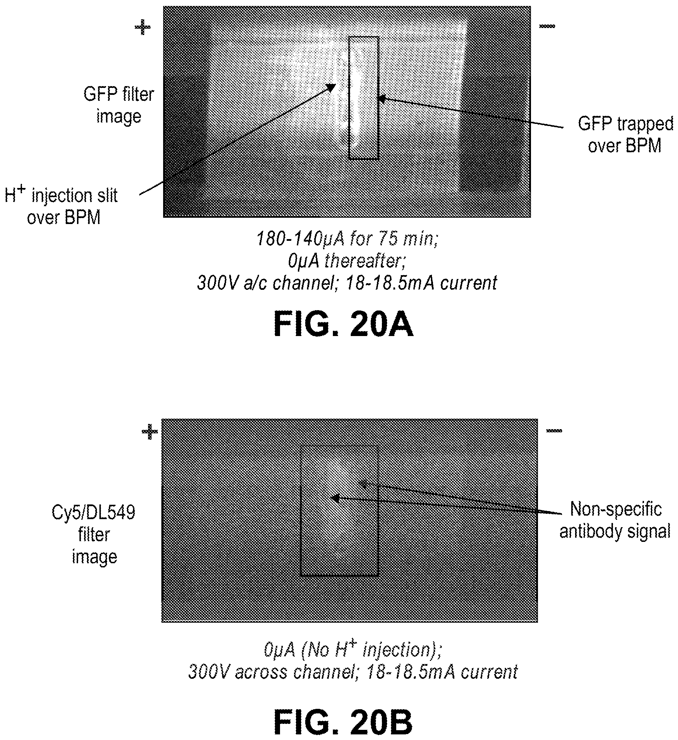

Figure 20A represents green fluorescent protein (GFP) signal following isoelectric focusing and adherence to chamber via prolonged H⁺ injection in buffer in a chamber. Figure 20B illustrates CY5 signal following precipitation/adherence of the GFP and electrophoretic introduction of a CY5-labeled anti-rabbit (non-specific) antibody.

### DEFINITIONS

An "affinity agent" refers to a molecule that specifically binds a target molecule. Exemplary affinity agents include, e.g., an antibody, antibody fragment, or aptamer. IN situations in which a target molecule is nucleic acid, the affinity agent can be, for example, a complementary nucleic acid.

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (e.g., as commonly used in an ELISA), biotin, digoxigenin, or haptens and proteins or other entities which can be made detectable, *e.g.,* by incorporating a radiolabel into the peptide or used to detect antibodies specifically reactive with the peptide. The labels may be incorporated into, e.g., antibodies and/or other proteins at any position. Any method known in the art for conjugating the antibody to the label may be employed, e.g., using methods described in Hermanson, Bioconjugate Techniques 1996, Academic Press, Inc., San Diego. Alternatively, methods using high affinity interactions may achieve the same results where one of a pair of binding partners binds to the other, e.g., biotin, streptavidin. Thus, for example, an affinity agent can be directly labeled with isotopes, chromophores, lumiphores, chromogens, or indirectly labeled such as with biotin to which a streptavidin complex (optionally including, e.g., a fluorescent, radioactive, or other moiety that can be directly detected) may later bind. Thus, a biotinylated antibody is considered a "labeled antibody" as used herein.

The phrase "specifically (or selectively) binds " or "specifically (or selectively) immunoreactive with" or "having binding specificity for", when referring to an affinity agent and target molecule, refers to a binding reaction between the affinity agent and target molecule which is determinative of the presence of the target molecule in the presence of a heterogeneous population of proteins and/or other biologics. Thus, for example, under immunoassay conditions, antibodies bind to a particular protein and do not bind in a significant amount to other proteins present in the sample. Specific binding to an antibody under such conditions may require an antibody that is selected for its specificity for a particular protein. For example, antibodies raised against a protein can be selected to obtain antibodies specifically immunoreactive with that protein and not with other proteins. A variety of immunoassay formats may be used to select antibodies specifically immunoreactive with a particular protein. For example, solid-phase ELISA immunoassays, Western blots, or immunohistochemistry are routinely used to select monoclonal antibodies specifically immunoreactive with a protein. *See,* Harlow and Lane Antibodies, A Laboratory Manual, Cold Spring Harbor Publications, NY (1988) for a description of immunoassay formats and conditions that can be used to determine specific immunoreactivity. Typically, a specific or selective reaction will be at least twice the background signal or noise and more typically more than 10 to 100 times background.

The term "biological sample" encompasses a variety of sample types obtained from an organism. The term encompasses bodily fluids such as blood, saliva, serum, plasma, urine and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, sedimentation, or enrichment for certain components. The term encompasses a clinical sample, and also includes cells in cell culture, cell supernatants, cell lysates, serum, plasma, other biological fluids, and tissue samples. The term is not limited to human-derived, or medical-related samples, and thus can include, e.g., plant-based, prokaryotic-based, or other samples of biological origin.

The term "antibody" refers to a polypeptide comprising a framework region (e.g., from an immunoglobulin gene), or fragments thereof, that specifically bind and recognize an antigen or desired target. Typically, the "variable region" contains the antigen-binding region of the antibody (or its functional equivalent) and controls specificity and affinity of binding. See Paul, Fundamental Immunology (2003).

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

An "isotype" is a class of antibodies defined by the heavy chain constant region. Immunoglobulin genes include the kappa, lambda, alpha, gamma, delta, epsilon, and mu constant region genes. Light chains are classified as either kappa or lambda. Heavy chains are classified as gamma, mu, alpha, delta, or epsilon, which in turn define the isotype classes, IgG, IgM, IgA, IgD and IgE, respectively.

Antibodies can exist as intact immunoglobulins or as any of a number of well-characterized fragments that include specific antigen-binding activity. Such fragments can be produced by digestion with various peptidases. Pepsin digests an antibody below the disulfide linkages in the hinge region to produce F(ab)'_{2,} a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (*see* Fundamental Immunology (Paul ed., 3d ed. 1993). While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized *de novo* using recombinant DNA methodologies (*e.g.,* single chain Fv) or those identified using phage display libraries (*see, e.g.,* McCafferty et al., Nature 348:552-554 (1990)).

"Target analyte" or "target molecule" can include a biomolecule, or molecule of biological origin. Target molecules include, but are not limited to, proteins, polynucleotides, metabolites, viruses, and virus-like particles and cells. Examples of proteins include but are not limited to antibodies, enzymes, growth regulators, clotting factors, and phosphoproteins. Examples of polynucleotides include DNA and RNA. Examples of viruses include enveloped and non-enveloped viruses.

The terms "nucleic acid" and "polynucleotide" are used interchangeably herein to refer to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, and peptide nucleic acids (PNAs).

The terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers.

In the claims appended hereto, the term "a" or "an" is intended to mean "one or more." The term "comprise" and variations thereof such as "comprises" and "comprising," when preceding the recitation of a step or an element, are intended to mean that the addition of further steps or elements is optional and not excluded.

### DETAILED DESCRIPTION OF THE INVENTION

### I. Introduction

As described in more detail herein, methods are provided that allow for detection of target molecules in samples in an apparatus using 1) electrical fields to move charged affinity agents and/or target molecules combined with 2) electronic control of pH of solution in proximity to sub-areas using proton or hydroxide injectors. The methods take advantage of the pH-dependence of charge of molecules, for example allowing for localization of charged molecules to a particular sub-area by setting the pH of solution in proximity to the sub-area to a pH at or close to the pI of the molecule of interest. At a molecule's pI, the molecule becomes uncharged and therefore does not move further in an electric field This is further described particular below.

The apparatus can have a variety of configurations. In particular, the apparatus comprises at least one chamber having a first and second electrode and at least two distinct sub-areas in the chamber. A "sub-area" refers to a region of the container at which molecules can be localized and in some aspects detected. Thus, for sub-areas at which detection is to occur, the sub-area can be sufficiently narrow or small to allow accurate determination of the quantity of molecules localized to that sub-area. For sub-area(s) in which detection does not occur (e.g., where a sample is initially positioned in the chamber), the sub-area(s) can be larger. Generally, the chamber will contain an aqueous solution compatible with the sample and affinity agents used. Different sub-areas do not overlap. In some embodiments, each sub-area represents less than about 50%, 40%, 30%, 20%, 10%, 5%, or 1% of the area of the chamber.

The terms "chamber" and "channel" are used synonymously. The terms encompass containers that are considerably (e.g., 10x, 100x, 1000x) longer than wide, which allow for multiple injectors along the long axis of the chamber. Without intending to limit the scope of the invention, it is noted that chambers of the following dimensions have been constructed:

| **Channel L/H/W in mm** | **Slit L/H/W In mm** | **Material** | **Channel volume (Vc; in µl)** | **Slit volume in µl (Vs; in µl)** |
|---|---|---|---|---|
| 90 X 0.3 X 3 | 3 X 0.5 X 0.3 | Glass/PMMA | 81 | 0.45 |
| 36 X 0.2 X 1 | 1 X 0.2 X 0.2 | COC | 7.2 | 0.04 |
| 221 X 0.25 X 1 | 1 X 0.25 X 0.2 | PMMA | 55 | 0.05 |
| 36 X 0.15 X .5 | .5 X 0.1 X 0.1 | PMMA | 2.7 | 0.005 |
| 33.6 X 0.25 X 1 | 1 X 0.25 X 0.23 | PMMA | 8.4 | 0.0575 |
| 221 X 0.25 X 1 | 1 X 0.25 X 0.2 | PMMA | 55 | 0.05 |

"Slits" refer to the size of the hole in the chamber through which the proton or hydroxide injector is connected to the chamber. A bipolar membrane at the slit divides the chamber from the injector.

The orientation of the electrodes (i.e., which is a cathode and which an anode) will depend on the charge of the molecules to be moved in the solution and the direction the molecules are to be moved. For example, a positively-charged molecule moves towards a cathode and a negatively-charged molecule moves towards an anode when an electrical voltage difference is present through the solution in the chamber between the cathode to the anode.

Generally, the electrodes should be oriented so that they are as close to each other as possible, i.e., directly across from each other. While other configurations are contemplated and possible, voltage and resistance increases as a function of distance.

Electrodes in the chamber can in some circumstances interfere and/or bind target molecules (e.g., protein) in the chamber. Thus, the electrodes can be separated from the chamber by a membrane or gel, thereby preventing target molecules from binding the electrodes.

The size and shape of the chamber can vary. While the chamber is depicted as a tube or channel (i.e., longer between the electrodes than across other axis), other configurations are also possible.

Current isoelectric focusing based protein/peptide fractionation technologies suffer from two main shortcomings. First, samples are separated over a fixed or limited pH range resulting in non-optimal fractionation of various samples. Second, pH gradients required for sample fractionation are established via chemicals (ampholytes) resulting in contamination of fractionated samples with chemicals and (potential) interference of downstream analysis. Dynamically adjustable pH 'step/s' spanning the pH range of ∼2-12 (can be further extended or contracted as needed) can be generated within a chamber filled with suitable buffers using digital pH technology. An example of such a gradient is displayed in Figure 16A. Complex mixtures of suitably buffered proteins (or peptides) will be electrophoresed within chamber so as to 'capture' proteins (or peptides) at their respective isoelectric points onto an affinity binding medium in either a single pH step (see Figure 16B) or multiple pH 'step/s' spanning the desired pH range. Subsequently, electronic adjustment of H⁺/OH⁻ generation at (each) 'step' will be used to 'release' simplified mixtures of ampholyte-free, charged species towards a harvesting chamber for collection and downstream analysis. *See, e.g.* Figure 16C. The release of the bound analyte may also optionally be performed by using a release agent (for example but not limited to denaturing solution or competitive binding reagent). This approach enables optimized fractionation of various protein/peptide samples (via adjusting protein/peptide capture and release in a sample-dependent manner) without contamination by chemical ampholytes.

Methods and devices combining proton/hydroxide injectors with affinity agents (e.g., antibodies) are disclosed. It is possible to take advantage of the concentration effect of isoelectric focusing/IEF. For example, protein solubility is lowest at/near its isoelectric point/pI. Thus, at or near pI for a target protein within an IEF chamber having a pH step gradient (see, e.g., Figure 17), proteins will concentrate into/adjacent to proton injection openings (e.g., 'slits') and precipitate. This concentrated precipitate can be used for immuno-detection of proteins of interest (POI), which in some cases can be post-translationally modified (PTM) variants from diverse biological samples. An advantage of this approach is higher resolution (owing to optimal separation via digital pH) and increased sensitivity (owing to concentration of proteins at their pI).

In particular, a protein(s) of interest (POI) can be focused in close proximity to high capacity beads or other solid support with affinity ligand (e.g., an antibody). This is exemplified, for example, in Figure 17. After capture of the antigen on the beads or other solid support the sample is washed to remove the unbound material from the chamber or vessel. Subsequently, the pH can be changed using the proton/hydroxide injector, thereby denaturing the affinity ligand (e.g., an antibody) and releasing the POI. The POI can be eluted for collection using either a "capture and release" pH gradient or via an electrophoretic gradient or simple elution by washing with an elution reagent such as a denaturing solution or solution containing competitive binding species. Optionally, affinity ligand (e.g., antibody) can be rejuvenated in the vessel by readjustment of the pH so that the antibody resumes its native state. By enclosing the system, the antibody can be reused multiple times with multiple samples for antigen purification. Such methods are useful for preparative methods of purifying proteins. The affinity ligand in some implementations may be an antibody, a metal chelate, for instance, for capturing poly-His tagged proteins, a glutathione for capturing GST tagged proteins, an oligo for capturing specific nucleic acids, an aptamer or other ligands that may have affinity interactions with biomolecules.

Because antibody-antigen interactions are pH dependent, first, a pH step is created in close proximity to the antibody, bringing the POI or other target analyte in proximity to the antibody. Subsequently, the pH step gradient is replaced with a pH plateau suited to enable antibody-antigen binding. Subsequently, washing can occur to remove unbound components of the solution.

Methods and devices combining proton/hydroxide injectors with affinity agents (e.g., antibodies) are disclosed. In particular, it is possible to take advantage of the concentration effect of isoelectric focusing/IEF, For example, protein solubility is lowest at/near its isoelectric point/pI. Thus, at or near pI for a target protein within an IEF chamber having a pH step gradient, proteins will concentrate into/adjacent to proton injection 'slits' and some will precipitate and/or adhere to the chamber. This concentrated precipitate can be used for immuno-detection of proteins of interest (POI), which in some cases can be post-translationally modified (PTM) variants from diverse biological samples. An advantage of this approach is higher resolution (owing to optimal separation via the creation of step gradients using the proton/hydroxide injectors), increased sensitivity (owing to concentration of proteins at their pI) as well as optimization of the pH for the interaction.

In particular, the method comprises the following steps. During step 1, proton injection in an IEF chamber will be used to precipitate POI/PTM variants (e.g., unphosphorylated versus hyperphosphorylated target proteins are 'separated' into openings (e.g., slits) wherein they each remain adhered via interaction with bipolar membrane/IEF chamber. In step 2, polyclonal primary antibodies injected into the IEF chamber will be reacted against POI (e.g., a phosphorylated target protein). In step 3, secondary antibodies coupled with horseradish peroxidase or alkaline phosphatase are introduced similarly as in step 2. In step 4, chemiluminescent or other labeling subtrates aree introduced into the IEF chamber and emitted signal (e.g., light) is detected. Alternatively, step 4 can be omitted. For example, fluorescently labeled secondary antibodies can be used making step 4 unnecessary. In this embodiment, the unique pI of the antibody/antigen complex could be used instead of a detection reagent in order to purify the complex of interest. While the above discussion is in the context of distinguishing post-translational modifications (PTMs), this method is not limited to PTM differentiation. For example, the methods can be used to detect the amount of any POI in a sample and may also use monoclonal antibodies as any or all antibody binding steps.

Prior immunodetection methods are performed by adding sample containing the target molecule (antigen) to antibody immobilized on a surface or a bead and waiting for diffusion to take place in order the antigen to come in close proximity to the antibody so the binding can occur. The process is time consuming since there is no specific driving force to bring the antigen in close proximity to the antibody. Also in some cases, the samples are complex (blood, serum, plasma, saliva, urine, lysates) and there are many other proteins present that may non-specifically bind to the antibody or to the surface around it. By using the proton/hydroxide injector technology these two issues are addressed by specifically driving the antigen to the immobilized antibody and concentrating it in close proximity to the antibody, as well as removing portion of the other proteins present in the sample and therefore minimizing the non-specific binding that may occur. *See, e.g.,* Figure 18. This will speed up the interaction, as well as deliver higher quality results with less non-specific interactions contributing to the signal.

In particular, the protein sample is separated in three fractions (step A, Figure 18):
- The proteins with pI higher than the pI of the target protein (antigen) are isoelectrically focused in the region of bipolar membrane (91) where a pH step encompassing pH higher than the pI of the antigen is created.
- The protein of interest is focused in the region of bipolar membrane (92) by creating a narrow pH range step encompassing the pI of the antigen
- The proteins with pI lower than the pI of the antigen are isoelectrically focused in the region of bipolar membrane (93) where pH step with range below the pI of the antigen is created

This way the antigen is captured and concentrated in the area of the binding antibody. In particular, the conditions are selected to prevent precipitation of the target analyte once the target arrives at its pI. For example, the solution in the chamber comprises a sufficient amount of a non-ionic detergent or other agent (e.g., organic solvent(s)) to promote solubility of the target analyte.

Because antibody-antigen interactions are pH dependent, first, a pH step is created in close proximity to the antibody, bringing the antigen in proximity to the antibody. Subsequently, the pH step gradient is replaced with a pH plateau suited to enable antibody-antigen binding.

The amount of the antigen bound to the antibody can be detected as desired. For example, the antibody can be immobilized on a sensor capable of detecting binding events (such as SPR, nano wire or other sensor types) or the antigen can be detected by performing sandwich type assay such as ELISA by using second antibody that is specific to the antigen but binds to a domain different than the domain that the immobilized antibody binds. In some implementations the sample may be pre-labeled, so the bound target analyte is detected after binding to the affinity ligand specific to this analyte.

Additional step (B) may be performed if needed (by applying the appropriate electric field) to further move the antigen to the surface (95) where the antibody is immobilized. The proposed methodology can work with various molecules as long as they have isoelectric point. For instance, instead of antibody, other binding ligands may be used, such as for example other proteins, peptides, DNA, and small molecules (including but not limited to aptamers).

In the method depicted in Figure 1, the sample is applied to the solution in the chamber and at least some components of the sample are allowed to localize in the first sub-area. Localization to the first sub-area can be achieved, for example, by tagging components of the sample prior to applying the sample to the chamber, and then localizing the tagged components by linking an affinity agent to the first sub-area where the affinity agent binds the tag. This is depicted in Figure 1, in which small filled circles represent the tag and large crosses represent the affinity agent. As one example, the tag can be biotin and the affinity agent that binds biotin can be avidin or streptavidin. Biotinylation of the sample is not specific for the target molecule in the sample and thus other components of the sample will also be localized to the first sub-area. Following localization, the solution can be changed and the chamber washed, thereby removing sample components that are not localized via the affinity agent to the first sub-area.

In another method (e.g., depicted in Figure 2), the target molecule in the sample can be selectively localized to the first sub-area of the chamber by an affinity agent linked to the first-sub-area, where the affinity agent specifically binds to the target molecule. For example, the affinity agent can be an antibody that specifically binds the target molecule. In this method, the target molecule is the primary or only component of the sample localized to the first sub-area due to the target's affinity for the affinity agent. As in the methoddescribed with reference to Figure 1, following localization, the chamber may be washed, thereby removing sample components that are not localized via the affinity agent to the first sub-area.

In another method, the sample is linked directly to a solid surface in the chamber (e.g., to a membrane in the chamber), thereby localizing components of the sample to a sub-area of the chamber. Components can be linked to the solid surface as desired. For example, the sample can be immobilized to a protein binding membrane (e.g., nitrocellulose affixed to the chamber). The sample can be covalently linked with the use of a cross linker such as formaldehyde, EDC or others. This is depicted in Figure 5.

Following localization of some or all components of the sample to the first sub-area, a detectably-labeled affinity agent that specifically binds the target molecule is added to the chamber and incubated under conditions to allow for binding of the affinity agent to the target molecule, if present. For instance, many antibodies will bind to their respective target molecule at a pH of ∼7-8. In particular, the detectably-labeled affinity agent may be an antibody. Following incubation, excess affinity agent is washed away, thereby leaving detectably-labeled affinity agent specifically bound to the localized target molecule. This method is shown in the second panel of Figure 1 and Figure 2.

The conditions in the chamber can be subsequently changed to elute the detectably-labeled affinity agent from the target molecule. The conditions can be changed, for example, by changing the pH of the solution by adding base or acid or changing the solution completely to replace the solution with a solution having a different pH or salt concentration to elute the detectably-labeled affinity agent. In particular, acidic (e.g., 1-2) pH or basic (e.g., 10-12) pH can be used to elute target molecule from the affinity agent (e.g., antibody). In particular, one or more proton or hydroxide injectors can be used to electronically change the pH of the solution in the chamber or at least in the solution in proximity to the first sub-area. Figures 1 and 2 depict a method in which the portion of the chamber having the first sub-area comprises an proton or hydroxide injector. A more detailed description of proton or hydroxide injectors is provided below.

The conditions can also be applied such that the eluted detectably-labeled affinity agent has a desired charge. For example, the overall charge of the affinity agent will be negative if the pH of the solution is above the pI of the affinity agent and the overall charge of the affinity agent will be positive if the pH of the solution is below the pI of the affinity agent. Once the charge of the affinity agent has the desired charge, a voltage difference can be applied across the electrodes, thereby generating an electric field that moves the charged affinity agents in the solution towards the appropriate electrode (cathode or anode depending on charge of the affinity agent).

In one method the detectably-labeled affinity agent is moved in solution to the location of an electrode, where the affinity agent is detected and/or quantified. Alternatively, the detectably-labeled affinity agent can be localized to a second sub-area of the chamber by setting the pH of the solution in proximity to the second sub-area to a pH at or close to the pI of the detectably-labeled affinity agent. The pH of the solution in proximity to the second sub-area can be controlled, for example, by inclusion of one or more proton or hydroxide injectors at the second sub-area. While not intending to be limited to a particular mechanism of action, it is believed that the pH of the solution in proximity to the second sub-area need not be exactly the pI of the detectably-labeled affinity agent but may merely be close to the pI to substantially eliminate overall charge of the detectably-labeled affinity agent, thereby stopping further movement of the detectably-labeled affinity agent. *See,* the third panel of Figures 1 and 2.

Once the detectably-labeled affinity agent is positioned at the electrode or at the second sub-area, the presence or quantity of the detectably-labeled affinity agent is detected. Detection of the detectably-labeled affinity agent will depend on the nature of the label. For example, if the label is a fluorescent dye, an optical detector set to measure signal at the appropriate wave length of the fluorescent dye can be used for detection. Quantity of the detectably-labeled affinity agent present will be proportional to the amount of target molecule in the original sample. Actual quantity of target molecule can be determined, if desired, using one or more standards and interpolation analysis.

The method described above (e.g., as depicted in Figures 1 and 2) can be modified to detect multiple target molecules in parallel (multiplexed). Representative multiplexing embodiments are depicted in Figures 6, 7, and 8. In some methods, at least some components of the sample are localized to a first sub-area of the chamber as described above, contacted with a plurality (e.g., 2, 3, 4, 5, or more) different detectably-labeled affinity agents, wherein the different affinity agents specifically bind different targets and have different detectable labels such that the different labels can be distinguished (e.g., are fluorescent at different wavelengths), under conditions such that the affinity agents bind their respective targets if present. Excess unbound affinity agents is washed away and the conditions are changed to elute the affinity agent from the target molecules. A voltage difference is then applied across the electrodes, thereby generating an electric field that moves the eluted affinity agents to a second sub-area (for example, labeled "4" in Figure 5). As described elsewhere, in some methods, the eluted affinity agents accumulate at the second sub-area because the solution in proximity to the second sub-area has a pH at or close to the pI of the affinity agents. The pH of the solution in proximity to the second sub-area can be controlled by one or more proton or hydroxide injectors. If the different affinity agents have different pIs, the pH can be adjusted in series for each affinity agent, with quantification of signal from the separate affinity agents determined before the pH is changed to accommodate the next affinity agent. Quantity of different affinity agents at the second sub-area can be detected by detecting the different labels associated with the different affinity agents. This method is illustrated, for example, in Figure 5.

Alternatively, in some methods, at least some components of the sample are localized to a first sub-area of the chamber as described above, contacted with a plurality (e.g., 2, 3, 4, 5, or more) different detectably-labeled affinity agents, wherein the labeled affinity agents specifically bind different targets and have different pIs. The affinity agents in this method can have the same or different label. Excess unbound affinity agents is washed away and the conditions are changed to elute the affinity agent from the target molecules. A voltage difference is then applied across the electrodes, thereby generating an electric field that moves the eluted affinity agents to different sub-areas, where the different sub-area have solution at different pH corresponding to the pI of a particular affinity agent. *See,* for example, Figure 6, where "A", "B", and "C" represent antibodies with affinity to different targets. As described elsewhere, the eluted affinity agents accumulate at the different sub-areas because the solution in proximity to the different sub-areas has a pH at or close to the pI of a particular affinity agent. The pH of the solution in proximity to the sub-areas can be controlled by one or more proton or hydroxide injectors.

As depicted in Figure 7, the two types of multiplexing discussed above (multiplexing by different label or multiplexing by different pI of affinity agent) can be combined if desired. For example, different affinity agents having the same pI can be differentially labeled while other affinity agents having different pIs can be distinguished by pI. This aspect is depicted in Figure 7 where different antibodies are represented by numbers representing their pI. Other symbols (*, +) indicate affinity agents with different affinity. Thus, "2*" and "2+" represent different antibodies, have different labels (*,+) but the same pI (2). They are positioned to the same sub-area, having a pH of about 2, and are detected by detecting their different signals (* and +). In any of the multiplexing embodiments, 2, 3, 4, 5, 6, 7, 8, 9, or more different affinity agents (different labels, different pI, or a combination thereof) can be used. Figure 8 illustrates how this aspect can be performed in parallel for multiple samples.

A different method is depicted in Figure 3. In this method, the sample complexed with the detectably-labeled affinity agent specific for the target molecule is provided in the chamber. In particular, the detectably-labeled affinity agent and the sample can be mixed prior to addition to the chamber. Alternatively, the detectably-labeled affinity agent can be added before or after the sample is added to the chamber and then submitted to conditions to allow for binding of the detectably-labeled affinity agent to the target molecule, if present. The conditions will also be of an appropriate pH such that the detectably-labeled affinity agent bound to the target molecule is charged, i.e., the pH is higher or lower than the pI of the complex of the detectably-labeled affinity agent bound to the target molecule. In some embodiments, the conditions are also designed such that unbound detectably-labeled affinity agent is also charged, though the pI of the unbound detectably-labeled affinity agent will be different than the pI of the complex formed from the detectably-labeled affinity agent bound to the target molecule.

Subsequently, a voltage difference can be applied across the electrodes, thereby moving the charged complex formed from the detectably-labeled affinity agent bound to the target molecule towards the appropriate electrode (cathode or anode depending on charge of the complex). In one aspect the complex is moved in solution to the location of an electrode, where the complex is detected and/or quantified. Alternatively, the complex can be localized to a second sub-area of the chamber by setting the pH of the solution in proximity to the second sub-area to a pH at or close to the pI of the complex. The pH of the solution in proximity to the second sub-area can be controlled, for example, by inclusion of one or more proton or hydroxide injectors at the second sub-area. The quantity of the complex can then be detected with an appropriately-located detector(s) and correlated to the quantity of the target molecule in the sample.

In some methods, the unbound detectably-labeled affinity agent (i.e., the excess affinity agent lacking a target molecule "partner") is localized to a third sub-area (see, e.g., Figure 3, label 8) of the chamber by setting the pH of the solution in proximity to the third sub-area to a pH at or close to the pI of the unbound detectably-labeled affinity agent. As noted above, the pI of the unbound detectably-labeled affinity agent will be different from the pI complex and therefore the location of the second and third sub-areas can be located in distinct locations in the chamber, thereby allowing for separate detection and quantification of bound and unbound detectably-labeled affinity agent. The pH of the solution in proximity to the third sub-area can be controlled, for example, by inclusion of one or more proton or hydroxide injectors at the third sub-area. The quantity of the complex can then be correlated based on the ratio of bound to unbound detectably-labeled affinity agent, assuming the amount of starting detectably-labeled affinity agent is known. If desired, unbound target molecule can also be localized by the pI of the unbound target molecule to a fourth sub-area (labeled 9 in Figure 3).

In yet another method, the sample is applied to the solution in a chamber having in a first sub-area (e.g., label 3 in Figure 4). In this method, an affinity agent specific for the target molecule is linked to the first sub-area. A labeled competitor molecule that competes with a target molecule for binding to the affinity agent is bound to the affinity agent. The sample can moved to the first sub-area under conditions to allow for binding competition between any target molecule present in the sample and the labeled competitor molecule. The amount of labeled competitor molecule displaced from the affinity agent will be proportional to the quantity of target molecule present in the sample. Following displacement of the labeled competitor molecule, a voltage difference is applied between the electrodes in the chamber, thereby moving the displaced labeled competitor molecule to a location where the labeled competitor molecule can be detected. The location can be adjacent to an electrode, or can be a second sub-area (4 as depicted in Figure 4). In some embodiments, the detectably-labeled affinity agent can be localized to a second sub-area of the chamber by setting the pH of the solution in proximity to the second sub-area to a pH at or close to the pI of the labeled competitor molecule. The pH of the solution in proximity to the second sub-area can be controlled, for example, by inclusion of one or more proton or hydroxide injectors at the second sub-area. The labeled competitor molecule localized at the second sub-area can subsequently be detected.

The labeled competitor molecule can be any molecule that competes for binding to the affinity agent with the target molecule. In particular, the labeled competitor molecule may comprise the target molecule, or an antigenic fragment thereof, linked to a detectable label.

In another method, an affinity agent specific for the target molecule is linked to the first sub-area. A labeled competitor molecule that competes with a target molecule for binding to the affinity agent is bound to the affinity agent similar to as described above. However, in this method, instead of starting with the labeled competitor molecule bound to the affinity agent, a known amount of labeled competitor molecule is mixed with the sample. The labeled competitor molecule can be mixed with the sample prior to addition of the sample to the solution in the chamber. Alternatively, the sample and labeled competitor molecule can be added to the solution in the chamber and allowed to mix prior to moving the sample and labeled competitor molecule to the first-sub-area. Once moved into proximity of the first sub-area and the linked affinity agents attached thereto, the sample and labeled competitor molecule are submitted to conditions to allow for binding of target molecules in the sample, as well as labeled competitor molecule to the affinity agents. The remaining unbound labeled competitor molecule can then be moved to a second sub-area by submitting a voltage difference to the electrodes, thereby moving the charged labeled competitor molecule in an electrical field. As described elsewhere herein, the unbound labeled competitor molecule will stop at the second sub-area when the solution in proximity to the second sub-area has a pH at or close to the pI of the unbound labeled competitor molecule. The pH of the solution in proximity to the second sub-area can be controlled, for example, by inclusion of one or more proton or hydroxide injectors (6) at the second sub-area. The unbound labeled competitor molecule at the second sub-area can then be detected and quantified. The amount of the unbound labeled competitor molecule, as well as the ratio of unbound labeled competitor molecule compared to the starting amount of labeled competitor molecule will be proportional to the amount of target molecules originally in the sample.

Another method is described with reference to Figure 9 and 10. In this method, the signal ultimately detected is a processed substrate of an enzyme linked to an affinity agent or a detachable label that is detached from the affinity agent. One initial format (Figure 9) for such aspects involves a chamber having an unlabeled affinity agent linked to the first sub-area (3). The sample is added under conditions to allow target molecules in the sample to bind the affinity agents, thereby localizing the target molecules to the first sub-area. In different initial format (one aspect of which is depicted in Figure 10), components of the sample, including the target molecule, if present, are bound to the first sub-area, either directly, or if the sample is biotinylated, via streptavidin or avidin linked to the first sub-area.

In either format, the result is that at least the target molecule is localized to the first sub-area (3). The solution in the chamber can optionally be washed to remove unbound components, and then contacted with an affinity agent comprising either an enzyme capable to altering a substrate to render it detectable (the "processed substrate) or a detectable label that is detachable from the affinity agent. In either case, the affinity agent can be used to bind to the immobilized target molecule in the first sub-area, or the labeled affinity agent can act as a secondary affinity agent with a primary affinity agent specifically binding the target molecule and the labeled secondary affinity agent binding to the primary affinity agent. An example of this latter option is the use of a mouse primary antibody to specifically bind to the target molecule and a labeled goat anti-mouse antibody as the secondary antibody. Once the labeled affinity agent is bound (directly or indirectly) to the localized target molecule, unbound molecules can be optionally washed away.

In the case of an enzyme-linked affinity agent, the substrate of the enzyme can be added under conditions such that the substrate is processed by the enzyme, thereby generating processed detectable substrate. Examples of possible enzyme/substrate pairs include, but are not limited to, horseradish peroxidase (substrates can include but are not limited to: 3,3'-diaminobenzidine (DAB), 3,3',5,5'-tetramethylbenzidine (TMB), 2,2'-Azinobis [3-ethylbenzothiazoline-6-sulfonic acid]-diammonium salt (ABST), or o-phenylenediamine dihydrochloride (OPD)), alkaline phosphatase (substrates can include but are not limited to: combination of nitro blue tetrazolium chloride (NBT) and 5-bromo-4-chloro-3-indolyl phosphate (BCIP) p-Nitrophenyl Phosphate, Disodium Salt (PNPP)), glucose oxidase (substrates can include but are not limited to: NBT), β-galactosidase (substrates can include but are not limited to: 5-bromo-4-chloro-3-indoyl-β-D-galactopyranoside (BCIG or X-Gal)), and luciferase (substrates include luciferin). The quantity of processed substrate should be proportional to the amount of target molecule in the sample.

In one method the processed substrate can be moved in solution to the location of an electrode, where the processed substrate is detected and/or quantified. Alternatively, the processed substrate can be localized to a second sub-area of the chamber by setting the pH of the solution in proximity to the second sub-area to a pH at or close to the pI of the processed substrate. The pH of the solution in proximity to the second sub-area can be controlled, for example, by inclusion of one or more proton or hydroxide injectors (6 in Figures 9 and 10) at the second sub-area (4). The processed substrate can then be detected and quantified.

In other methods, the affinity agent comprises a label that can be displaced from the affinity agent. "Can be displaced," as used in this context, means that the conditions within the chamber can be changed to specifically release the label from the affinity agent. For example, in some embodiments, the affinity agent is linked to the detectable label via a double stranded nucleic acid (e.g., dsDNA, dsRNA, or a mimetic thereof), wherein one strand is linked to the affinity agent and one strand is linked to the label. Under appropriate conditions, the double-stranded nucleic acid can be denatured, thereby displacing the label from the affinity agent. Denaturation conditions can comprise, for example, a change in the pH of the solution in proximity to the affinity agent. The change in pH can be achieved, for example, using one or more proton or hydroxide injectors in proximity to the first sub-area.

### II. Methods

As is clear from the descriptions above, methods for detecting and/or quantifying one or more target molecule in a biological or other sample can be achieved using the apparatus described herein.

Samples can be any type of sample potentially comprising a target molecule of interest that can be bound by an affinity agent. In some embodiments, the sample is a biological sample. The target molecule refers to a molecule of interest to be detected or quantified. Target molecules can include proteins, polynucleotides (e.g., DNA or RNA), viruses, and metabolites. Examples of target proteins include but are not limited to antibodies, enzymes, growth regulators, and clotting factors.

"Affinity agents" as described herein refer to any agents (e.g., molecules) that specifically bind to an intended target. An exemplary affinity agent is an antibody (e.g., a monoclonal antibody)or fragment thereof with antigen binding specificity. Further, a number of different synthetic molecular scaffolds can be used to display the variable light and heavy chain sequences of antibodies specific for the target molecule. Moreover, random libraries of peptides, aptamers, or other molecules can be used to screen for affinity agents with specificity to a particular target molecule. A publication describing use of the fibronectin type III domain (FN3) as a specific molecular scaffold on which to display peptides including CDRS is Koide, A. et al. J. Mol. Biol 284:1141 1151(1988). Other scaffolding alternatives include, e.g., "minibodies" (Pessi, A. et al., Nature 362:367 369 (1993)), tendamistat (McConnell, S. J. and Hoess, R. H. J. Mol. Biol, 250:460 470 (1995)), and "camelized" VH domain (Davies J. and Riechmann, L. BiolTechnology 13:475 479 (1995)). Other scaffolds that are not based on the immunoglobulin like folded structure are reviewed in Nygren, P. A. and Uhlen, M. Curr. Opin. Struct. Biol. 7:463 469 (1997). U.S. Pat. No. 6,153,380 describes additional scaffolds. The term "affinity agents" encompasses molecules comprising synthetic molecular scaffolds such as those described above to display binding domains with a binding specificity for the target molecule.

The specificity of antibody binding can be defined in terms of the comparative dissociation constants (Kd) of the antibody for the target molecule as compared to the dissociation constant with respect to the antibody and other materials in the environment or unrelated molecules in general. Typically, the Kd for the antibody with respect to the unrelated material will be at least 2-fold, 3-fold, 4-fold, 5-fold, 10-fold, 20-fold, 50-fold, 100-fold, 200-fold or higher than Kd with respect to the target.

The labels used can be any label that is capable of directly or indirectly emitting or generating detectable signal. In some embodiments, the labels are fluorophores. A vast array of fluorophores are reported in the literature and thus known to those skilled in the art, and many are readily available from commercial suppliers to the biotechnology industry. Literature sources for fluorophores include Cardullo et al., Proc. Natl. Acad. Sci. USA 85: 8790-8794 (1988); Dexter, D.L., J. of Chemical Physics 21: 836- 850 (1953); Hochstrasser et al., Biophysical Chemistry 45: 133-141 (1992); Selvin, P., Methods in Enzymology 246: 300-334 (1995); Steinberg, I. Ann. Rev. Biochem., 40: 83- 114 (1971); Stryer, L. Ann. Rev. Biochem., 47: 819-846 (1978); Wang et al., Tetrahedron Letters 31: 6493-6496 (1990); Wang et al., Anal. Chem. 67: 1197-1203 (1995).

The following is a list of examples of fluorophores:
4-acetamido-4'-isothiocyanatostilbene-2,2'disulfonic acid
acridine
acridine isothiocyanate
5-(2'-aminoethyl)aminonaphthalene-1-sulfonic acid (EDANS)
4-amino-N-[3-vinylsulfonyl)phenyl]naphthalimide-3,5 disulfonate
N-(4-anilino-1-naphthyl)maleimide
anthranilamide
BODIPY
Brilliant Yellow
coumarin
7-amino-4-methylcoumarin (AMC, Coumarin 120)
7-amino-4-trifluoromethylcoumarin (Coumaran 151)
cyanine dyes
cyanosine
4',6-diaminidino-2-phenylindole (DAPI)
5', 5"-dibromopyrogallol-sulfonaphthalein (Bromopyrogallol Red)
7-diethylamino-3-(4'-isothiocyanatophenyl)-4-methylcoumarin
diethylenetriamine pentaacetate
4,4'-diisothiocyanatodihydro-stilbene-2,2'-disulfonic acid
4,4'-diisothiocyanatostilbene-2,2'-disulfonic acid
5-[dimethylamino]naphthalene-1-sulfonyl chloride (DNS, dansylchloride)
4-(4'-dimethylaminophenylazo)benzoic acid (DABCYL)
4-dimethylaminophenylazophenyl-4'-isothiocyanate (DABITC)
eosin
eosin isothiocyanate
erythrosin B
erythrosin isothiocyanate
ethidium
5-carboxyfluorescein (FAM)
5-(4,6-dichlorotriazin-2-yl)aminofluorescein (DTAF)
2',7'-dimethoxy-4'5'-dichloro-6-carboxyfluorescein (JOE)
fluorescein
fluorescein isothiocyanate
fluorescamine
IR144
IR1446
Malachite Green isothiocyanate
4-methylumbelliferone
ortho cresolphthalein
nitrotyrosine
pararosaniline
Phenol Red
phycoerythrin (including but not limited to B and R types)
o-phthaldialdehyde
pyrene
pyrene butyrate
succinimidyl 1-pyrene butyrate
quantum dots
Reactive Red 4 (Cibacron™ Brilliant Red 3B-A)
6-carboxy-X-rhodamine (ROX)
6-carboxyrhodamine (R6G)
lissamine rhodamine B sulfonyl chloride rhodamine
rhodamine B
rhodamine 123
rhodamine X isothiocyanate
sulforhodamine B
sulforhodamine 101
sulfonyl chloride derivative of sulforhodamine 101 (Texas Red)
N,N,N' ,N' -tetramethyl-6-carboxyrhodamine (TAMRA)
tetramethyl rhodamine
tetramethyl rhodamine isothiocyanate (TRITC)
riboflavin
rosolic acid
lanthanide chelate derivatives

If desired, the fluorophores (or other labels) can be used in combination, with a distinct labels for affinity agents with different target specificities (e.g., for multiplexing). In some embodiments, however, a single label is used for all labeled affinity agents, the assays being differentiated solely by differentiation based on pI.

The attachment of any of these fluorophores to affinity agents can be achieved by conventional covalent bonding, using appropriate functional groups on the fluorophores and on the affinity agents. The recognition of such groups and the reactions to form the linkages will be readily apparent to those skilled in the art.

As noted elsewhere herein, the methods can achieve detection of the presence or absence of a particular target molecule(s) in a sample. In some embodiments, the approximate quantity of the target molecule in the sample can be determined, for example as explained elsewhere herein.

### III. Proton and hydroxide injectors

A proton or hydroxide "injector " refers to one or more compartments, separated from a sub-chamber or other vessel (e.g., such as a reservoir), by an opening or "slit" and divided by a bipolar membrane(s), in wherein the compartment(s) contain an electrode(s). Depending on the orientation of the electric field (e.g., orientation of the anode and cathode) in the compartment(s), the compartment(s) can be designed to inject protons or hydroxide ions through the bipolar membrane(s) and into the adjacent chamber.

By controlling the current and configuration, one can thereby control the pH of solution in the chamber in proximity to the proton or hydroxide injector. Generally, it can be desirable to increase the surface area of the bipolar membrane as this allows for decreased electrical resistance.

The membrane(s) "divides" the compartments from the chamber by forming a barrier that separates solution in a compartment from the chamber, e.g., at least to the level of solution in the chamber. For example, in embodiments in which the chamber is open at the top (or alternatively, has a top cover that can be removed), the membrane(s) can be designed to completely divide a compartment from the chamber at least up to the level of solution in the chamber and/or compartment, or to a level designated as a maximum for solution loading. As desired, the membranes can be designed to be higher than the solution level so as to avoid accidental transfer (e.g., splashing) from one portion to another. If desired, the membranes can be "framed" by a solid material (e.g., plastic) or otherwise anchored between the chamber and the compartment.

The electrodes can be formed from any conducting or semi-conducting substance. For example, in some embodiments, one or more electrode comprises a metal. In some embodiments, the metal is zinc, copper, or platinum. For example, the electrodes can be platinum or can be platinum-plated. Generally, maximal surface area for electrodes is desirable. A flattened electrode, for example, provides more surface area than a wire.

WO2009/027970 describes methods and devices (i.e., proton or hydroxide injectors) useful in producing local concentrations of protons or hydroxide ions, proton or hydroxide concentration gradients, and desired proton or hydroxide concentration topographies in an environment, such as an electrolyte solution, and a gel. WO2011/021195 and WO2011/021196 describe methods and devices for isoelectric focusing using proton/hydroxide injectors and also describes display of data.

Proton/hydroxide injector technology can be used to affect the pH of the solution in a chamber, or at least the solution in the chamber in proximity to the injector. Briefly, the proton/hydroxide injector may comprise a compartment adjacent to the apparatus chamber, with an electrode inside the compartment, and a bipolar membrane separating the compartment from the channel. *See, e.g.,* Figures 11-12. A bipolar membrane is an ionexchange membrane having a structure in which a cation-exchange membrane and an anion-exchange membrane are joined together, and allows for water molecules to be split into protons and hydroxide ions. Voltage applied between the compartment and the channel divided by the bipolar membrane leads to water splitting and injection of protons or hydroxide ions into the channel. Some advantages of this technology can include, for example, bubble-free water hydrolysis and injection of generated ions directly to the channel, allowing short response time (e.g., if desired, below 1 minute).

By applying the appropriate voltage to the electrodes in the chamber an electric field across the solution in the chamber is generated and charged molecules move accordingly. In some embodiments, the charged molecules can be added in proximity to the anode or cathode in the chamber (in which the pH is controlled at least in part by a proton injector or a hydroxide injector), and subsequently the voltage is applied, thereby delivering the charged molecule to a desired position in the chamber at a time determined by the user.

The direction of movement of the molecule will depend on the charge of the molecule and the polarity of the applied voltage.

Systems incorporating the above-describedapparatus are also disclosed. Systems can include, for example, a power supply and power regulator to control current and/or voltage to electrodes in the chamber and/or injectors. *See, e.g.,* Figure 14. Pumps for regulating flow of liquids, a mechanism for stirring or mixing solution in the chamber, and heating or cooling units can be included. In particular, the system may include a pH and/or conductivity probe in the chamber. Generally, it can be desirable to place the probe at a distance from the electric field lines between electrodes to improve readings.

### EXAMPLE

### Example 1: Separation of Target(s) Based on pI

Two fluorescently-labeled peptides, one with a pI of 5.0, one with a pI of 6.8, were placed into a chamber comprising a pH 8.5 phosphate buffer. The chamber comprises two proton injectors, with the first proton injector having a current applied of 150 µA and the second proton injector having a current applied of 65 µA, thereby generating separate localized areas within the solution having different pH. In view of the higher current, the first injector generated a more acidic pH in the area of the chamber near the first injector compared to the pH near the second injector. An electric field was generated across the chamber, thereby moving charged molecules according to their charge. The pI 6.8 peptides focused on the area near the first proton injector and the pI 5.0 peptides focused on the area of the chamber near the second proton injector. This shows that molecules having different pI can be moved and isolated in different areas of a solution in a chamber using electronic control of their movement in combination with localization based on control of local pH in the solution using ion injectors.

### Example 2: Precipitation/Trapping of Target(s) Based on pI

This experiment shows that some target molecules precipitate/adhere to an IEF channel when positioned at their pI, and that the resulting precipitated targets can subsequently be detected. Green Fluorescent Protein (GFP, 1 mg) and human saliva (1,5 mg) were combined with STB 8.5 (4 mM each Sodium Citrate, Sodium Phosphate, Sodium Pyrophosphate, and 13 mM Sodium Sulfate, pH 8.5) and the resulting mixture was introduced into a chamber comprising a proton injector. The injector was set to generate a pH step at approximately the pI of GFP (∼5.4) and voltage was run through the first and second electrodes across the chamber, thereby electrophoresing GFP through the chamber and up to the pH gradient, where GFP stopped due to lack of charge. GFP was trapped by isoelectric focusing over a bipolar membrane (BPM) and H⁺ injection.

While not true for all targets, GFP precipitated/adhered to an IEF channel at the pH step. The voltage was then shut off. As shown in Figure 19A and 20A, which detects GFP fluorescence, GFP localized at the pH step. Subsequently, an anti-GFP antibody (DL649) labeled with Cy5 was introduced to the chamber and electrophoresed for 60 min across channel and over the GFP precipitate. Signal under a Cy5 filter shows that the anti-GFP antibody localized with the GFP (Figure 19B), demonstrating that this system detects target molecules that are localized in a pH step gradient. In contrast, Figure 20B displays results from a parallel experiment using a non-specific anti-rabbit antibody. Only background signal was observed from the non-specific antibody.

## Claims

1. A method of detecting a target analyte, the method comprising,
providing into a chamber a sample comprising a mixture of molecules including one or more target analytes, the chamber comprising a first and a second electrode, wherein a first and second sub-areas of the compartment are between the electrodes;
generating a pH gradient in the chamber with one or more proton and/or hydroxyl injectors;
applying a current across the chamber, thereby positioning analytes in the chamber at the first sub-area based on the isoelectric point (pI) of the analytes, wherein the target analyte is precipitated and/or adhered to the chamber once positioned at the first sub-area in the chamber corresponding to the target analyte pI;
contacting the target analyte with a labeled affinity agent that specifically binds the target analyte;
applying a current between the first and second electrode thereby moving the affinity agent that bound to the target analyte, if any, to a second sub-area of the compartment, wherein the solution in proximity to the second sub-area has a pH
controlled by one or more proton/hydroxide injector(s), and detecting the presence of the affinity agent bound to the target analyte at the second sub-area of the compartment; and
detecting the precipitated analyte.

2. The method of claim 1, further comprising:
moving labeled affinity agent not bound to the target analyte to a third location, wherein solution in proximity to the third sub-area has a pH at approximately the pI of the labeled affinity agent not bound to the target analyte, wherein the pH of the solution in proximity to the third sub-area is controlled by the one or more proton and/or hydroxyl injector(s); and
detecting the presence of the labeled affinity agent not bound to the target analyte.

3. The method of claim 1, further comprising:
between the contacting and the applying, washing away labeled affinity agent not bound to the target analyte; and
eluting the labeled affinity agent bound to the target analyte such that the labeled affinity agent no longer binds the target analyte when the labeled affinity agent is moved to the second sub-area of the chamber.

4. The method of claim 1, wherein the target analyte is moved to a third sub-area of the chamber, wherein solution in proximity to the third sub-area has a pH at approximately the pI of the unbound target analyte, wherein the pH is controlled by the one or more proton and/or hydroxyl injector(s), and wherein the target analyte is collected.

5. The method of claim 1, wherein the labeled affinity agent is an antibody.

6. The method of claim 1, wherein the target analyte is a protein.

7. The method of claim 1, wherein the target analyte is a post-translationally-modified protein.

## Patentansprüche

1. Verfahren zum Nachweisen eines Zielanalyten, wobei das Verfahren umfasst:
Bereitstellen einer Probe, die ein Gemisch von Molekülen umfasst, das einen oder mehrere Zielanalyten aufweist, in einer Kammer, wobei die Kammer eine erste und eine zweite Elektrode umfasst, wobei sich ein erster und ein zweiter Teilbereich des Kompartiments zwischen den Elektroden befindet;
Erzeugen eines pH-Gradienten in der Kammer mit einem oder mehreren Protonen- und/oder Hydroxid-Injektoren;
Anlegen einer Spannung an die Kammer, wodurch Analyten in der Kammer auf der Basis des isoelektrischen Punkts (pI) der Analyten in dem ersten Teilbereich positioniert werden, wobei der Zielanalyt ausgefällt und/oder an die Kammer geheftet wird, sobald er in der Kammer im ersten Teilbereich entsprechend dem pI des Zielanalyten positioniert ist;
In-Kontakt-Bringen des Zielanalyten mit einem markierten Affinitätsmittel, das spezifisch an den Zielanalyten bindet;
Anlegen einer Spannung zwischen der ersten und zweiten Elektrode, wodurch das gegebenenfalls an den Zielanalyten gebundene Affinitätsmittel in einen zweiten Teilbereich des Kompartiments bewegt wird, wobei die Lösung in der Nähe des zweiten Teilbereichs einen pH-Wert aufweist, der durch einen oder mehrere Protonen/Hydroxid-Injektoren gesteuert ist, und Nachweisen der Anwesenheit des an den Zielanalyten gebundenen Affinitätsmittels im zweiten Teilbereich des Kompartiments; und
Nachweisen des ausgefällten Analyten.

2. Verfahren gemäß Anspruch 1, weiterhin umfassend:
Bewegen von markiertem Affinitätsmittel, das nicht an den Zielanalyten gebunden ist, an eine dritte Stelle, wobei die Lösung in der Nähe des dritten Teilbereichs einen pH-Wert aufweist, der ungefähr dem pI des markierten Affinitätsmittels, das nicht an den Zielanalyten gebunden ist, entspricht, wobei der pH-Wert der Lösung in der Nähe des dritten Teilbereichs durch den einen oder die mehreren Protonen- und/oder Hydroxid-Injektoren gesteuert ist; und
Nachweisen der Anwesenheit des markierten Affinitätsmittels, das nicht an den Zielanalyten gebunden ist.

3. Verfahren gemäß Anspruch 1, weiterhin umfassend:
zwischen dem In-Kontakt-Bringen und dem Anlegen: Wegwaschen von markiertem Affinitätsmittel, das nicht an den Zielanalyten gebunden ist; und
Eluieren des markierten Affinitätsmittels, das an den Zielanalyten gebunden ist, so dass das markierte Affinitätsmittel nicht mehr an den Zielanalyten bindet, wenn das markierte Affinitätsmittel in den zweiten Teilbereich der Kammer bewegt wird.

4. Verfahren gemäß Anspruch 1, wobei der Zielanalyt in einen dritten Teilbereich der Kammer bewegt wird, wobei die Lösung in der Nähe des dritten Teilbereichs einen pH-Wert aufweist, der ungefähr dem pI des ungebundenen Zielanalyten entspricht, wobei der pH-Wert durch den einen oder die mehreren Protonen- und/oder Hydroxid-Injektoren gesteuert ist und wobei der Zielanalyt aufgefangen wird.

5. Verfahren gemäß Anspruch 1, wobei das markierte Affinitätsmittel ein Antikörper ist.

6. Verfahren gemäß Anspruch 1, wobei der Zielanalyt ein Protein ist.

7. Verfahren gemäß Anspruch 1, wobei der Zielanalyt ein posttranslational modifiziertes Protein ist.

## Revendications

1. Procédé de détection d'un analyte cible, le procédé comprenant,
la fourniture dans une chambre d'un échantillon comprenant un mélange de molécules incluant un ou plusieurs analytes cibles, la chambre comprenant une première et une seconde électrode, dans lequel un premier et un deuxième sous-secteur du compartiment se trouvent entre les électrodes ;
la génération d'un gradient de pH dans la chambre avec un ou plusieurs injecteurs de protons et/ou d'hydroxyde ;
l'application d'un courant à travers la chambre, positionnant ainsi des analytes dans la chambre au niveau du premier sous-secteur sur la base du point isoélectrique (pI) des analytes, dans lequel l'analyte cible est précipité et/ou mis à adhérer à la chambre une fois positionné au niveau du premier sous-secteur dans la chambre correspondant au pI de l'analyte cible ;
la mise en contact de l'analyte cible avec un agent d'affinité marqué qui se lie spécifiquement à l'analyte cible ;
l'application d'un courant entre la première et la seconde électrode, déplaçant ainsi l'agent d'affinité qui se lie à l'analyte cible, le cas échéant, vers un deuxième sous-secteur du compartiment, dans lequel la solution à proximité du deuxième sous-secteur a un pH contrôlé par un ou plusieurs injecteurs de protons et/ou d'hydroxyde, et la détection de la présence de l'agent d'affinité lié à l'analyte cible au niveau du deuxième sous-secteur du compartiment ; et
la détection de l'analyte précipité.

2. Procédé selon la revendication 1, comprenant en outre :
le déplacement d'un agent d'affinité marqué non lié à l'analyte cible vers un troisième emplacement, dans lequel une solution à proximité du troisième sous-secteur a un pH approximativement du niveau du pI de l'agent d'affinité marqué non lié à l'analyte cible, dans lequel le pH de la solution à proximité du troisième sous-secteur est contrôlé par les un ou plusieurs injecteurs de protons et/ou d'hydroxyde ; et
la détection de la présence de l'agent d'affinité marqué non lié à l'analyte cible.

3. Procédé selon la revendication 1, comprenant en outre :
entre la mise en contact et l'application, l'élimination par rinçage de l'agent d'affinité marqué non lié à l'analyte cible ; et
l'élution de l'agent d'affinité marqué lié à l'analyte cible de telle sorte que l'agent d'affinité marqué ne se lie plus à l'analyte cible lorsque l'agent d'affinité marqué est déplacé vers le deuxième sous-secteur de la chambre.

4. Procédé selon la revendication 1, dans lequel l'analyte cible est déplacé vers un troisième sous-secteur de la chambre, dans lequel la solution à proximité du troisième sous-secteur a un pH approximativement du niveau du pI de l'analyte cible non lié, dans lequel le pH est contrôlé par les un ou plusieurs injecteurs de protons et/ou d'hydroxyde, et dans lequel l'analyte cible est collecté.

5. Procédé selon la revendication 1, dans lequel l'agent d'affinité marqué est un anticorps.

6. Procédé selon la revendication 1, dans lequel l'analyte cible est une protéine.

7. Procédé selon la revendication 1, dans lequel l'analyte cible est une protéine modifiée après la traduction.
